# EUROPÄISCHE PATENTSCHRIFT

(11) **EP 0 835 934 B1**
(45) Veröffentlichungstag und Bekanntmachung des Hinweises auf die Patenterteilung: **30.06.2010**
(21) Anmeldenummer: 97117504.7
(22) Anmeldetag: 09.10.1997
(51) Int. Cl.: C12N 15/31, C12N 15/62, C07K 14/36, C12N 1/21, G01N 33/50

(54) **Streptavidinmuteine**
Muteins of streptavidin
Mutéines de streptavidine

(30) Priorität: 10.10.1996 DE 19641876
(43) Veröffentlichungstag der Anmeldung: 15.04.1998
(73) Patentinhaber: IBA GmbH, 37079 Göttingen (DE)
(72) Erfinder: Skerra, Arne, Prof,-Dr., 64283 Darmstadt (DE); Voss, Selma, 55218 Ingelheim (DE)
(74) Vertreter: Viering, Jentschura & Partner

(56) Entgegenhaltungen:
- EP-A- 0 799 890
- DE-A- 4 237 113
- VOSS S ET AL: "Mutagenesis of a flexible loop in streptavidin leads to higher affinity for the Strep-tag II peptide and improved performance in recombinant protein purification." PROTEIN ENGINEERING, (1997 AUG) 10 (8) 975-82. JOURNAL CODE: PR1. ISSN: 0269-2139., ENGLAND: UNITED KINGDOM, XP002107709

### SEQUENZPROTOKOLL

(1) ALLGEMEINE ANGABEN:
   (i) ANMELDER:
      (A) NAME: Institut fuer Bioanalytik IBA
      (B) STRASSE: Rudolf-Wissell-Strasse 28
      (C) ORT: Goettingen
      (E) LAND: Deutschland
      (F) POSTLEITZAHL: D-37079
   (ii) BEZEICHNUNG DER ERFINDUNG: Streptavidinmuteine
   (iii) ANZAHL DER SEQUENZEN: 15
   (iv) COMPUTER-LESBARE FASSUNG:
      (A) DATENTRGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LNGE: 9 Aminosuren
      (B) ART: Aminosure
      (C) STRANGFORM: Einzelstrang
      (D) TOPOLOGIE: linear
   (ii) ART DES MOLEKLS: Peptid
   (xi) SEQUENZBESCHREIBUNG: SEQ ID NO: 1: 
Ala Trp Arg His Pro Gln Phe Gly Gly
1               5
(2) ANGABEN ZU SEQ ID NO: 2:
   (i) SEQUENZKENNZEICHEN:
      (A) LNGE: 8 Aminosuren
      (B) ART: Aminosure
      (C) STRANGFORM: Einzelstrang
      (D) TOPOLOGIE: linear
   (ii) ART DES MOLEKLS: Peptid
   (xi) SEQUENZBESCHREIBUNG: SEQ ID NO: 2: 
Trp Ser His Pro Gln Phe Glu Lys
1               5
(2) ANGABEN ZU SEQ ID NO: 3:
   (i) SEQUENZKENNZEICHEN:
      (A) LNGE: 12 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 3:
      GAGTCGGCCG TC 12
(2) ANGABEN ZU SEQ ID NO: 4:
   (i) SEQUENZKENNZEICHEN:
      (A) LNGE: 4 Aminosuren
      (B) ART: Aminosure
      (C) STRANGFORM: Einzelstrang
      (D) TOPOLOGIE: linear
   (ii) ART DES MOLEKLS: Peptid
   (xi) SEQUENZBESCHREIBUNG: SEQ ID NO: 4: 
Glu Ser Ala Val
1
(2) ANGABEN ZU SEQ ID NO: 5:
   (i) SEQUENZKENNZEICHEN:
      (A) LNGE: 12 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 5:
      GTCACGGCGC GT 12
(2) ANGABEN ZU SEQ ID NO: 6:
   (i) SEQUENZKENNZEICHEN:
      (A) LNGE: 4 Aminosuren
      (B) ART: Aminosure
      (C) STRANGFORM: Einzelstrang
      (D) TOPOLOGIE: linear
   (ii) ART DES MOLEKLS: Peptid
   (xi) SEQUENZBESCHREIBUNG: SEQ ID NO: 6: 
Val Thr Ala Arg
1
(2) ANGABEN ZU SEQ ID NO: 7:
   (i) SEQUENZKENNZEICHEN:
      (A) LNGE: 12 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 7:
      ATCGGTGCGA GG 12
(2) ANGABEN ZU SEQ ID NO: 8:
   (i) SEQUENZKENNZEICHEN:
      (A) LNGE: 4 Aminosuren
      (B) ART: Aminosure
      (C) STRANGFORM: Einzelstrang
      (D) TOPOLOGIE: linear
   (ii) ART DES MOLEKLS: Peptid
   (xi) SEQUENZBESCHREIBUNG: SEQ ID NO: 8: 
Ile Gly Ala Arg
1
(2) ANGABEN ZU SEQ ID NO: 9:
   (i) SEQUENZKENNZEICHEN:
      (A) LNGE: 34 Basenpaare
      (B) ART: Nucleotid
      (C) STRANGFORM: Einzelstrang
      (D) TOPOLOGIE: linear
      (ii) ART DES MOLEKLS: Sonstige Nucleinsure
   (xi) SEQUENZBESCHREIBUNG: SEQ ID NO: 9:
      GAGATACAGC TGCAGAAGCA GGTATCACCG GCAC 34
(2) ANGABEN ZU SEQ ID NO: 10:
   (i) SEQUENZKENNZEICHEN:
      (A) LNGE: 37 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 10:
      CGGATCAAGC TTATTAGGAG GCGGCGGACG GCTTCAC
(2) ANGABEN ZU SEQ ID NO: 11:
   (i) SEQUENZKENNZEICHEN:
      (A) LNGE: 74 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 11: 
TCGTGACCGC GGGTGCAGAC GGAGCTCTGA CCGGTACCTA CNNSNNKNNS NNKGGCAACG 60

CC[?]AGAGCCG CTAC 74
(2) ANGABEN ZU SEQ ID NO: 12:
   (i) SEQUENZKENNZEICHEN:
      (A) LNGE: 37 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 12:
      CGGATCAAGC TTATTAGGAG GCGGCGGACG GCTTCAC 37
(2) ANGABEN ZU SEQ ID NO: 13:
   (i) SEQUENZKENNZEICHEN:
      (A) LNGE: 42 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 13:
      TAATGTTCTA GAACATGGAG AAAATAAAGT GAAACAAAGG AC 42
(2) ANGABEN ZU SEQ ID NO: 14:
   (i) SEQUENZKENNZEICHEN:
      (A) LNGE: 31 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 14:
      GCTAGGCGGT TTCAGCCCCA GAGCGGCTTT C 31
(2) ANGABEN ZU SEQ ID NO: 15:
   (i) SEQUENZKENNZEICHEN:
      (A) LNGE: 54 Basenpaare
      (B) ART: Nucleotid
      (C) STRANGFORM: Einzelstrang
      (D) TOPOLOGIE: linear
   (ii) ART DES MOLEKLS: Sonstige Nucleinsure
   (xi) SEQUENZBESCHREIBUNG: SEQ ID NO: 15:
      CACAGGTCAA GCTTATTATT TTTCGAACTG CGGGTGAGAC CAAGCGCTGC CTGC 54

## Beschreibung

Die vorliegende Erfindung betrifft Streptavidinmuteine, Verfahren zur Herstellung derartiger Proteine über rekombinante DNA-Technologie, sowie die Verwendung dieser Streptavidinmuteine zur Isolierung, Reinigung bzw. Bestimmung von biologischen Substanzen, insbesondere von anderen rekombinanten Proteinen.

Das System Biotin/Streptavidin ist ein heutzutage in der Molekularbiologie allgemein bekanntes Bindungssystem, dessen Bedeutung in den letzten Jahren erheblich zugenommen hat und welches in unterschiedlichen Anwendungsbereichen eingesetzt wird. Dabei macht man sich die spezifische Affinität zwischen Biotin und Streptavidin zunutze, die gleichzeitig mit einer Affinitätskonstante in der Größenordnung von 10¹³ eine der stabilsten bekannten nichtkovalenten Wechselwirkungen ist.

Wichtige herkömmliche Anwendungen bestehen in diversen Abtrennungs- und Nachweisverfahren unter Verwendung meist biotinylierter Enzyme oder/und Antikörper in unterschiedlichen Variationen. Beispiele sind z.B. ELISA, Western Blot etc. Eine Voraussetzung für derartige Verfahren besteht darin, daß das im Verfahren in biotinylierter Form verwendete Reagenz bzw. Enzym zunächst in Reinform erhalten werden kann, um die Biotinylierung, die in einer chemischen Reaktion erfolgt, durchführen zu können.

Für bestimmte Anwendungen ist jedoch eine Biotinylierung nicht oder zumindest nicht in einfacher Weise möglich, wie beispielsweise beim Nachweis und bei der Aufreinigung rekombinant hergestellter Proteine, die zuvor noch nicht isoliert wurden. In der Vergangenheit wurde daher nach Wegen gesucht, das Biotin/Streptavidin-System zu modifizieren, um seinen Anwendungsbereich zu erweitern.

Ein erfolgreicher Ansatz bestand in der Herstellung von Peptidliganden, die ebenfalls eine spezifische Bindungsaffinität zu Streptavidin aufweisen. Geeignete Peptidliganden und entsprechende Fusionsproteine sind in DE-OS 42 37 113 offenbart. Der Vorteil dieser Peptidliganden im Vergleich zu Biotin besteht im wesentlichen darin, daß deren kodierende Sequenz auf DNA-Ebene mit dem Gen eines gewünschten Proteins verknüpft und anschließend zusammen mit der des Proteins koexprimiert werden kann, wodurch ein mit dem Peptidliganden markiertes, d.h. an ihn fusioniertes, rekombinantes Protein gebildet wird. Aufgrund der geringen Größe der Peptidliganden und der Tatsache, daß sie an den N- oder C-Terminus des gewünschten Proteins angehängt werden können, d.h. also in Bereichen, die für die Struktur und biochemische Funktion eines Proteins häufig keine große Bedeutung haben, ist es im allgemeinen auch nicht notwendig, nach der Isolierung und vor Verwendung des Proteins zu anderen Zwecken den Peptidliganden wieder abzuspalten, so daß auch diesbezüglich eine günstige Verfahrensökonomie gegeben ist. In der Tat ist bisher kein Fall bekannt geworden, bei dem eine Abspaltung erforderlich gewesen wäre. Sollte dennoch die Notwendigkeit der Abspaltung bestehen, kann dies durch Inserierung einer Proteasespaltstelle zwischen Bindungspeptid und Proteinsequenz bewerkstelligt werden.

Geeignete derartige Peptidliganden sind ausführlich beschrieben, beispielsweise bei Schmidt und Skerra, Protein Eng. 6 (1993), 109-122 und J. Chromatogr. A 676 (1994), 337-345 sowie bei Schmidt et al., J. Mol. Biol. 255 (1996), 753-766.

Vorteile des Streptavidin-Peptidligand-Systems bestehen darin, daß die Reinigung rekombinanter Proteine überhaupt möglich wird und daß diese Reinigung z.B. durch Affinitätschromatographie unter sehr milden Elutionsbedingungen erfolgen kann, da der gebundene Peptidligand als Teil des rekombinanten Proteins kompetitiv von Biotin bzw. dessen Derivaten verdrängt wird. Außerdem gelingt über den Peptidliganden der Nachweis des rekombinanten Proteins beispielsweise durch Western Blot, ELISA oder durch Immunmikroskopie mittels geeigneter Streptavidinkonjugate.

Ein Nachteil dieses Systems bestand bisher in einer relativ niedrigen Affinität. Für den Komplex zwischen Streptavidin und dem als Strep-tag bezeichneten Peptidliganden (AWRHPQFGG) wurde mittels isothermer Titrationskalorimetrie eine Affinitätskonstante von 2,7 · 10⁴ M⁻¹ bestimmt. Obwohl es Hinweise gab, daß die Bindung für ein den Peptidliganden enthaltendes Fusionsprotein etwas stärker sein könnte, ist es wünschenswert, über ein System mit prinzipiell verbesserter Affinität zu verfügen.

Die der Erfindung zugrundeliegende Aufgabe bestand somit darin, das System Streptavidin/Peptidligand hinsichtlich der Bindungsstärke zu optimieren.

Nach anfangs durchgeführten Versuchen, die Sequenz des Peptidliganden weiter zu optimieren, mußte davon ausgegangen werden, daß die Peptidliganden gemäß DE-OS-4237113 offensichtlich bereits ein Optimum darstellten und dieser Ansatz somit wenig erfolgversprechend war.

Mit der Verfügbarkeit der Kristallstruktur des Streptavidin/Peptidligand-Komplexes in hoher Auflösung konnte zwar ein besseres Verständnis der molekularen Wechselwirkungen bzw. der strukturellen Merkmale gewonnen werden (Schmidt et al. 1996), supra), es konnte aus diesen Strukturdaten jedoch keine klare Information erhalten werden ob und in welcher Weise eine Modifizierung der Peptidsequenz oder des Streptavidins in rationaler Weise durchgeführt werden kann, um die Affinität zu verbessern und somit die eingangs gestellte Aufgabe zu lösen. In einem evolutiven Forschungsansatz wurde nun überraschenderweise festgestellt, daß eine Verbesserung der Bindungsaffinität für das Streptavidin/Peptidligand-System durch Mutation im Bereich der Aminosäurepositionen 44 bis 53 von Streptavidin erreicht werden kann.

In diesem Zusammenhang sei die Europäische Patentanmeldung 0 799 890 erwähnt. Diese Patentanmeldung ist bezogen auf die vorliegende Anmeldung nach Art. 54(3)(4) EPÜ für die gemeinsam benannten Vertragsstaaten Deutschland, Frankreich und Italien zu berücksichtigen ist und offenbart Muteine des Streptavidins mit einer verringerten Bindungsaktivität für Biotin. Explizit werden in der Patentanmeldung EP 0 799 890 folgende Streptavidin-Muteine offenbart, die die Aminosäuresequenz von Ala 13 bis Ala 138, bezogen auf die Aminosäuresequenz von Wildtyp-Streptavidin, besitzen und bei denen
1) an Sequenzposition 45 die Aminosäure Arg,
2) an Sequenzposition 25 die Aminosäure Trp und an Sequenzposition 45 die Aminosäure Arg,
3) an Sequenzposition 45 die Aminosäure Trp und an Sequenzposition 110 die Aminosäure Trp,
4) an Sequenzposition 45 die Aminosäure Tyr und an Sequenzposition 110 die Aminosäure Trp,
5) an Sequenzposition 25 die Aminosäure Trp, an Sequenzposition 45 die Aminosäure Trp und an Sequenzposition 110 die Aminosäure Trp,
6) an Sequenzposition 25 die Aminosäure Trp, an Sequenzposition 45 die Aminosäure Tyr und an Sequenzposition 110 die Aminosäure Trp,
7) an Sequenzposition 27 die Aminosäure Arg, an Sequenzposition 45 die Aminosäure Arg und an Sequenzposition 110 die Aminosäure Trp,
8) an Sequenzposition 27 die Aminosäure Arg, an Sequenzposition 45 die Aminosäure Arg und an Sequenzposition 120 die Aminosäure Ala oder
9) an Sequenzposition 27 die Aminosäure Arg und an Sequenzposition 45 die Aminosäure Arg vorhanden ist.
²

Ein Gegenstand der vorliegenden Erfindung ist somit ein Polypeptid, ausgewählt aus Muteinen von Streptavidin, welches dadurch gekennzeichnet ist, daß es (a) innerhalb des Bereichs der Aminosäure-Positionen 44 bis 53, bezogen auf die Aminosäuresequenz von Wildtyp-(wt)-Streptavidin (Nomenklatur gemäß Argarana et al., Nucleic Acids Res. 14 (1986), 1871-1882), mindestens eine Mutation, insbesondere einen Aminosäureaustausch enthält und (b) eine höhere Bindungsaffinität für Peptidliganden, umfassend die Aminosäuresequenz Trp-X-His-Pro-Gln-Phe-Y-Z, wobei X eine beliebige Aminosäure darstellt und Y und Z entweder beide Gly oder Y Glu und Z Arg oder Lys bedeuten, aufweist als wt-Streptavidin.

Für die Vertragstaaten Deutschland, Frankreich und Italien sind dabei die 9 in EP 0 799 890 explizit genannten Muteine mittels eines Disclaimers vom Gegenstand des Anspruch 1 ausgenommen.

Außerhalb des Bereichs der Aminosäurepositionen 44 bis 53 können die Streptavidinmuteine der vorliegenden Erfindung der Aminosäuresequenz von wt-Streptavidin entsprechen. Andererseits kann die Aminosäuresequenz der erfindungsgemäßen Muteine auch außerhalb des Bereichs der Aminosäuren 44 bis 53 Unterschiede zur wt-Streptavidinsequenz aufweisen. Derartige Varianten der Streptavidinsequenz umfassen natürlich vorkommende sowie künstlich erzeugte Varianten und unter den Abweichungen werden Austausche, Insertionen, Deletionen von Aminosäureresten sowie N- oder/und C-terminale Additionen verstanden.

Die Angabe "höhere Bindungsaffinität" bezieht sich im Sinne der vorliegenden Anmeldung auf einen Komplex aus einem erfindungsgemäßen Streptavidinmutein und einem Peptidliganden gemäß DE-OS-4237113 und kann durch übliche Verfahren, z.B. ELISA, Fluoreszenztitration oder Titrationskalorimetrie bestimmt werden. Die auf diese Weise bestimmte Bindungsaffinität wird durch Kenngrößen angegeben, wie etwa Affinitäts- und Dissoziationskonstante oder thermodynamische Parameter. Die Erhöhung der Bindungsaffinität, die mit einem erfindungsgemäß innerhalb des Bereichs der Aminosäurepositionen 44 bis 53 veränderten Streptavidinmutein im Vergleich zum entsprechenden unveränderten Streptavidin erhalten wird, beträgt mindestens Faktor 5, bevorzugt mindestens Faktor 10 und stärker bevorzugt mindestens Faktor 20. Bevorzugte erfindungsgemäße Streptavidinmuteine umfassen mindestens eine Mutation im Bereich der Aminosäurepositionen 44 bis 47.

Bevorzugte erfindungsgemäße Streptavidinmuteine sind von Streptavidinvarianten abgeleitet, die am N- oder/und C-Terminus verkürzt sind. Besonders bevorzugt sind die aus dem Stand der Technik bekannten Minimal-Streptavidine, die sowohl N- als auch C-terminal verkürzt sind. Ein bevorzugtes Polypeptid gemäß der vorliegenden Erfindung umfaßt außerhalb des mutagenisierten Bereichs die Aminosäuresequenz eines Minimal-Streptavidins, das N-terminal im Bereich der Aminosäurepositionen 10 bis 16 beginnt und C-terminal im Bereich der Aminosäurepositionen 133 bis 142 endet. Besonders bevorzugt entspricht das Polypeptid außerhalb des Mutationsbereichs einem Minimal-Streptavidin, das eine Aminosäuresequenz von Position Ala¹³ bis Ser¹³⁹ umfaßt und gegebenenfalls einen N-terminalen Methioninrest aufweist. Die Numerierung von Aminosäurepositionen bezieht sich in dieser Anmeldung durchgehend auf die Numerierung von wt-Streptavidin (Argarana et al., Nucleic Acids Res. 14 (1986), 1871-1882).

Besonders bevorzugte erfindungsgemäße Streptavidinmuteine sind **dadurch gekennzeichnet, daß** an Position 44 Glu durch eine hydrophobe aliphatische Aminosäure, z.B. Val, Ala, Ile oder Leu, ersetzt ist, an Position 45 eine beliebige Aminosäure vorhanden ist, an Position 46 eine hydrophobe aliphatische Aminosäure vorhanden ist oder/und an Position 47 Val durch eine basische Aminosäure, z.B. Arg oder Lys, insbesondere Arg, ersetzt ist. Streptavidinmuteine, bei denen die hydrophobe aliphatische Aminosäure an Position 46 Ala ist, d.h. daß an tion 46 Ala ist, d.h. daß an Position 46 kein Austausch vorliegt, oder/und bei denen die basische Aminosäure an Position 47 Arg ist oder/und bei denen die hydrophobe aliphatische Aminosäure an Position 44 Val oder Ile ist, haben eine besonders hohe Affinität für den bei Schmidt et al., Supra, beschriebenen Peptidliganden mit der Sequenz WSHPQFEK (Strep-tag II).

Spezifische Beispiele für erfindungsgemäße Streptavidinmuteine besitzen im Bereich der Aminosäurepositionen 44 bis 47 die Sequenzen Val⁴⁴-Thr⁴⁵-Ala⁴⁶-Arg⁴⁷ oder Ile⁴⁴-Gly⁴⁵-Ala⁴⁶-Arg⁴⁷.

Aus praktischen Erwägungen ist es wünschenswert, über einen weiteren Liganden zu verfügen, der aufgrund einer höheren Bindungsaffinität oder/und bei Anwesenheit in höheren Konzentrationen die Bindung der vorstehend definierten Peptidliganden (gemäß DE-OS-4237113) zum erfindungsgemäßen Streptavidinmutein wieder auflösen kann. Auf diese Weise ist es möglich, einen gebundenen Peptidliganden bzw. Proteine, an die ein Peptidligand fusioniert ist, unter sehr milden Elutionsbedingungen freizusetzen. Unter diesem Aspekt betrifft die vorliegende Erfindung somit solche erfindungsgemäßen Streptavidinmuteine, deren Bindungsaffinität für Peptidliganden derart ist, daß sie kompetitiv durch andere Streptavidin-Liganden, z.B. Biotin, Iminobiotin, Liponsäure, Desthiobiotin, Diaminobiotin, HABA (Hdroxyazobenzolbenzoesäure) oder/und Dimethyl-HABA, wieder eluiert werden können. Die Verwendung von gefärbten Substanzen wie etwa HABA hat den Vorteil, daß die Elution visuell überprüft werden kann.

Ungeachtet dessen ist jedoch, wie vorstehend definiert, die Bindungsaffinität des Streptavidinmuteins für Peptidliganden höher als die des zugrundeliegenden wt-Streptavidins. Die Bindungsaffinität, ausgedrückt als Affinitätskonstante, ist somit bezogen auf den in SEQ ID NO. 1 dargestellten Peptidliganden AWRHPQFGG (im folgenden auch als Strep-tag bezeichnet) größer als 2,7 · 10⁴ M⁻¹ bzw. bezogen auf in SEQ ID NO. 2 dargestellten Peptidliganden WSHPQFEK (im folgenden auch als Strep-tag II bezeichnet) größer als 1,4 · 10⁴ M⁻¹, d.h. größer als die veröffentlichten Werte für die Komplexbildung der jeweiligen Peptidliganden mit wt-Streptavidin (innerhalb der Fehlergrenzen). Im allgemeinen liegt die Affinitätskonstante für das Strep-tag II mindestens um einen Faktor 10, bevorzugt um einen Faktor 10 bis 200 über den jeweiligen Werten für wt-Streptavidin.

Für bestimmte Nachweisverfahren kann es bevorzugt sein, die Streptavidinmuteine der vorliegenden Erfindung in markierter Form zu verwenden. Demgemäß ist ein weiterer Gegenstand dieser Erfindung ein erfindungsgemäßes Polypeptid, welches dadurch gekennzeichnet ist, daß es zumindest eine Markierung trägt. Geeignete Markierungsgruppen sind dem Fachmann bekannt und umfassen die üblichen Radio-, Fluoreszenz-, Lumineszenz- und Chromophormarkierungen sowie Substanzen bzw. Enzyme, die in einer chemischen oder enzymatischen Reaktion ein bestimmbares Substrat erzeugen. Dabei können alle für wt-Streptavidin bekannten Markierungen auch an die erfindungsgemäßen Streptavidinmuteine gekoppelt werden.

Gemäß einem weiteren Aspekt betrifft die vorliegende Erfindung eine Nukleinsäure, die eine für das Streptavidin kodierende Sequenz umfaßt. Eine derartige Nukleinsäure ist gegebenenfalls operativ mit einer für ein Signalpeptid kodierenden Sequenz verknüpft und in einer besonderen Ausführungsform ist die für das Signalpeptid kodierende Sequenz die Sequenz für das OmpA-Signalpeptid. Darüberhinaus können jedoch auch, insbesondere in Abhängigkeit des verwendeten Expressionssystems bzw. der Wirtszelle, andere Signalpeptide verwendet werden und sogar bevorzugt sein. Derartige Signalpeptide sind im Stand der Technik in großer Anzahl bekannt und werden hier nicht eingehend erläutert. Bevorzugt ist aber die cytoplasmatische Expression, d.h. mit einem Start-Methionin anstelle der Signalsequenz (vgl. Schmidt und Skerra (1994), supra).

Gemäß einem weiteren Aspekt betrifft die vorliegende Erfindung einen Vektor, der mindestens eine Kopie einer vorstehend genannten Nukleinsäure in operativ funktioneller Umgebung enthält. Unter einer operativ funktionellen Umgebung werden solche Elemente verstanden, die die Expression, d.h. Transkription oder/und eine nachfolgende Prozessierung der mRNA ermöglichen, begünstigen, erleichtern oder/und erhöhen. Beispiele für derartige Elemente sind etwa Promotoren, Enhancer, Transkriptionsinitiationsstellen und -terminationsstellen, Translationsinitiationsstellen, polyA-Stellen etc.

Der Vektor wird in Abhängigkeit vom vorgesehenen Expressionssystem ausgewählt und hierfür kommen sowohl single copy-Plasmide, multi copy-Plasmide als auch Vehikel, die eine Integration der Nukleinsäure in das Wirtsgenom begünstigen, in Betracht. Geeignete Vektoren sind in großer Anzahl aus dem Stand der Technik bekannt und werden hierin nicht ausführlich beschrieben. Sie enthalten gegebenenfalls übliche, für Vektoren verwendete Elemente, wie Resistenzen, Selektionsmarker oder/und Elemente, die z.B. eine Amplifizierung der Nukleinsäure oder die Induktion der Expression gestatten.

Gemäß einem weiteren Aspekt betrifft die vorliegende Erfindung eine Zelle, die mit einem derartigen Vektor, welcher als Insert mindestens eine Kopie einer für ein erfindungsgemäßes Streptavidinmutein kodierenden Nukleinsäuresequenz trägt, transformiert oder transfiziert ist. Die Auswahl der Zelle ist nicht sonderlich kritisch und im allgemeinen können beliebige Zellen, die für derartige Zwecke geeignet sind, verwendet werden. Es kommen sowohl prokaryontische als auch eukaryontische Zellen sowie Hefen in Betracht. Aus praktischen Gründen werden zur Expression eines unglykosilierten Proteins wie im vorliegenden Fall im allgemeinen prokaryontische Zellen bevorzugt und insbesondere E. coli.

Unter einem nochmals anderen Aspekt betrifft die vorliegende Erfindung ein Verfahren zur Herstellung eines erfindungsgemäßen Streptavidinmuteins, welches durch die folgenden Schritte gekennzeichnet ist:
(a) Transformieren einer geeigneten Wirtszelle mit einem Vektor, der eine für das Streptavidinmutein kodierende Nukleinsäure umfaßt,
(b) Kultivieren der Wirtszelle unter Bedingungen, bei denen eine Expression des Streptavidinmuteins erfolgt,
(c) Gewinnen des Polypeptids.

Bezüglich des Herstellungsverfahrens ist zu beachten, daß die erfindungsgemäßen Streptavidinmuteine aufgrund ihrer Bindungsfähigkeit mit dem zelleigenen Biotin eine toxische Wirkung ausüben können. Die Bedingungen beim Kultivieren der Wirtszelle sollten daher so gewählt werden, daß das entstehende Expressionsprodukt entweder mittels einer geeigneten Signalsequenz aus dem Inneren der verwendeten Wirtszelle z.B. in das Periplasma oder das Kulturmedium ausgeschleust wird oder in Form unlöslicher Inclusion bodies (Einschlußkörper) im Inneren der Zelle aggregiert. Im ersteren Fall kann das erfindungsgemäße Streptavidinmutein aus der periplasmatischen Zellfraktion oder dem Zellüberstand gewonnen werden, während im letzteren Fall Schritt (c) des erfindungsgemäßen Verfahrens die Lyse der Wirtszellen, die Gewinnung des Streptavidinmuteins in Form von Inclusion bodies und die Renaturierung des Streptavidinmuteins umfaßt. Als Wirtszelle ist in diesem Fall E. coli bevorzugt.

Die praktischen Anwendungsmöglichkeiten für die erfindungsgemäßen Streptavidinmuteine bzw. das System Streptavidinmutein/Peptidligand entsprechen im wesentlichen denjenigen der herkömmlichen Systeme Streptavidin/Biotin bzw. Streptavidin/ Peptidligand. Vorteile ergeben sich insbesondere in Situationen, bei denen eine höhere Bindungsstärke als zwischen nativem Streptavidin und Peptidligand erwünscht ist, bzw. in Situationen, bei denen eine Biotinylierung eines interessierenden Substrats nicht möglich oder ungünstiger zu bewerkstelligen ist als die entsprechende Verknüpfung mit einem Peptidliganden.

Die Vorzüge gegenüber dem herkömmlichen Streptavidin/Biotin-System kommen insbesondere bei der Affinitätschromatographie bzw. in Reinigungs-, Isolierungs- oder Bestimmungsverfahren für rekombinante Proteine zum Tragen. Demnach betrifft die Erfindung auch die Verwendung eines erfindungsgemäßen Streptavidinmuteins in einem Verfahren zur Isolierung, Reinigung oder zum Nachweis eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, wobei man eine das zu isolierende oder zu reinigende Protein enthaltende Flüssigkeit mit dem gegebenenfalls immobilisierten Streptavidinmutein unter geeigneten Bedingungen in Kontakt bringt, um eine Bindung der Peptidsequenz an das Streptavidinmutein zu bewirken, den resultierenden Komplex aus der Flüssigkeit abtrennt und das Protein aus dem Komplex freisetzt oder nachweist. Besonders vorteilhaft wird die Peptidsequenz in Form des Strep-tag oder Strep-tag II gewählt. Vorzugsweise ist die Peptidsequenz an den N- oder/und C-Terminus des Proteins fusioniert. Das Streptavidinmutein kann an eine Festphase gebunden oder mit ihr bindefähig sein.

Ein Vorzug bei der Verwendung des erfindungsgemäßen Systems Streptavidinmutein/Peptidligand in einem Isolierungs- oder Reinigungsverfahren besteht darin, daß zur Elution des den Peptidliganden tragenden Fusionsproteins sehr milde Bedingungen verwendet werden können. So kann eine mit dem Streptavidinmutein gekoppelte Festphase, wie etwa eine Affinitätschromatographiesäule, an welche das Fusionsprotein adsorbiert wurde, mit einer ausreichenden Konzentration eines Liganden, ausgewählt aus Biotin und Derivaten davon, inkubiert werden, um das Fusionsprotein aus dem Komplex freizusetzen. In diesem Zusammenhang hat sich die Verwendung von Desthiobiotin als besonders vorteilhaft herausgestellt.

Die Verwendung der erfindungsgemäßen Streptavidinmuteine in Nachweisverfahren kann im wesentlichen analog zu den entsprechenden Verfahren erfolgen, die für herkömmliches Streptavidin bekannt sind. Eine darüber hinausgehende Anwendungsmöglichkeit ergibt sich bei der qualitativen oder quantitativen Bestimmung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, wobei man das zu bestimmende Protein mit einem markierten Streptavidinmutein unter geeigneten Bedingungen, um eine Bindung der Peptidsequenz an das Streptavidinmutein zu bewirken, in Kontakt bringt und die Markierung bestimmt. Ein derartiges Bestimmungsverfahren kann beispielsweise qualitativ zum Nachweis von Proteinen in Western Blots oder quantitativ als ELISA durchgeführt werden. Geeignete Markierungen sind alle bekannten radioaktiven und nichtradioaktiven Markierungsgruppen, z.B. Lumineszenzgruppen, Enzyme, Metalle, Metallkomplexe etc. Das Streptavidin kann direkt z.B. durch kovalente Kopplung markiert sein. Aber auch indirekte Markierungen, z.B. markierte Anti-Streptavidin-Antikörper oder biotinylierte Enzyme usw. können eingesetzt werden.

Ein weiterer Gegenstand der Erfindung ist die Verwendung der erfindungsgemäßen Strepatividinmuteine zur Immobilisierung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist. Diese Immobilisierung erfolgt vorzugsweise an mit Streptavidinmuteinen beschichteten Festphasen wie etwa Mikrotiterplatten, Mikrobeads aus organischen oder aus paramagnetischen Materialien oder Sensorchips. Außerdem ist es natürlich auch möglich, die erfindungsgemäßen Streptavidinmuteine im herkömmlichen Streptavidin/Biotin(derivat) -System zu verwenden. In anderen Worten bedeutet dies die Verwendung der erfindungsgemäßen Streptavidinmuteine zur Bestimmung oder Gewinnung von Substanzen, die eine mit Streptavidin bindefähige Gruppe tragen. Sofern nur ein Teil des wt-Streptavidins durch die erfindungsgemäßen Streptavidinmuteine ersetzt wird, können dabei über die Ausbildung gemischter Tetramere nochmals besondere Wirkungen erzielt werden.

Gemäß einem nochmals weiteren Aspekt betrifft die Erfindung auch einen Reagenzienkit, der ein erfindungsgemäßes Streptavidinmutein sowie gegebenenfalls übliche Puffer-, Hilfs- und Zusatzstoffe umfaßt. Ein solcher Reagenzienkit ist insbesondere zur Verwendung in einem vorstehend beschriebenen Isolierungs-, Reinigungs- oder Bestimmungsverfahren vorgesehen. Der Kit ist jedoch auch für andere Verfahren geeignet, bei denen das herkömmliche Streptavidin/Biotin-System verwendet wird, z.B. für Nukleinsäurehybridisierungsassays oder Immunoassays. Der Reagenzienkit kann das erfindungsgemäße Streptavidinmutein in festphasengebundener oder/und markierter Form enthalten.

Die Erfindung wird weiter veranschaulicht durch die nachstehenden Beispiele und die beigefügten Zeichnungen, in denen:
- Figur 1: eine Schemazeichnung des Vektors pASK75-SAp dar- stellt;
- Figur 2: eine Graphik darstellt, welche die Bindungsaffinität von rekombinantem wt-Streptavidin im Vergleich zu erfindungsgemäßen Streptavidinmuteinen in einem ELISA zeigt;
- Figur 3: die Bindungsaffinität von rekombinantem wt-Strepta- vidin im Vergleich zu einem erfindungsgemäßen Strep- tavidinmutein in einer Fluoreszenztitration zeigt und
- Figur 4: die Reinigung eines Strep-tag-Fusionsproteins unter Verwendung eines Streptavidinmuteins durch Affini- tätschromatographie zeigt.

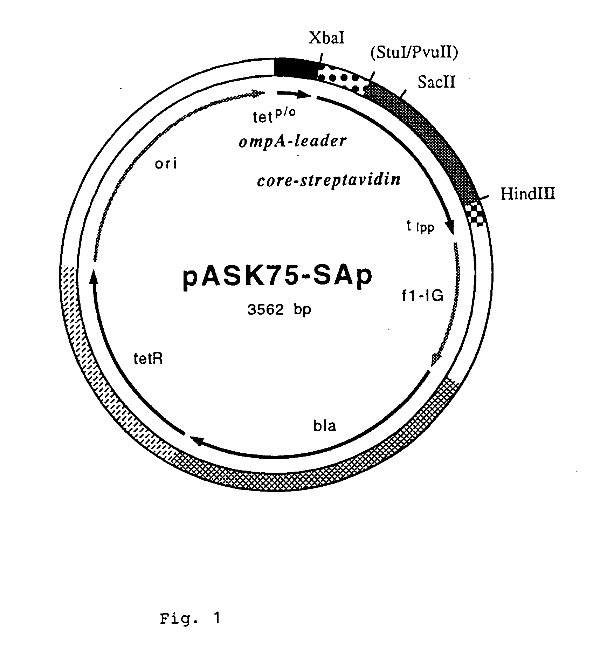

Figur 1 zeigt den Expressionsvektor pASK75-SAp, der eine für ein Minimal-Streptavidin (Ala¹³ bis Ser¹³⁹) kodierende Sequenz, eine für das OmpA-Signalpeptid kodierende Sequenz, sowie den Tetracyclin Promotor/Operator (tet^{P/O}) zur Transkriptionsregulation enthält.

Weitere gekennzeichnete Regionen des Vektors sind die intergenische Region des filamentösen Phagen f1 (f1-IG), der Replikationsursprung (ori), das β-Lactamasegen (bla) für die Ampicillinresistenz, das Tetracyclinrepressorgen (tetR) und der Lipoprotein-Transkriptionsterminator (t₁ₚₚ).

Das die kodierenden Sequenzen für Signalpeptid und Minimal-Streptavidin enthaltende hybride Strukturgen beginnt an der XbaI-Stelle und reicht stromabwärts bis zur HindIII-Stelle. Der Übergang zwischen Signalsequenz und Streptavidin erfolgt an der StuI/PvuII-Stelle. Die SacII-Stelle, die zum Inserieren der mutierten Streptavidingensequenzen verwendet wurde, ist ebenfalls angegeben.

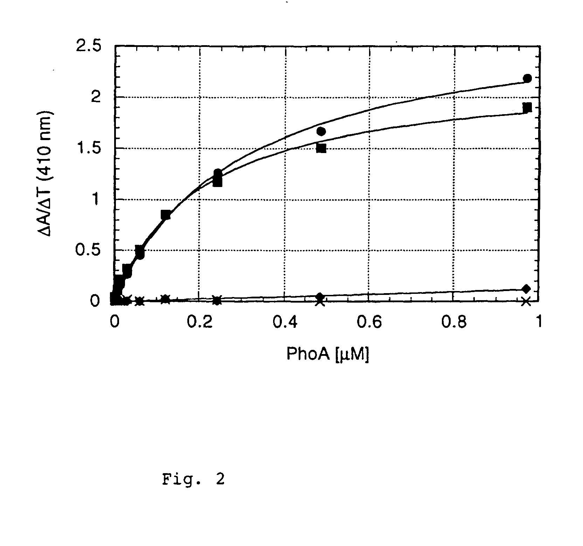

Figur 2 zeigt die verbesserte Affinität der erfindungsgemäßen Streptavidinmuteine für den Peptidliganden Strep-tag II in einem ELISA. Dazu wurden jeweils Reihen einer ELISA-Platte mit äquivalenten Konzentrationen eines rekombinanten wt-Streptavidin (Raute), des Muteins "1" (Kreis) oder "2" (Quadrat) beschichtet oder lediglich mit BSA (Kreuz) abgesättigt. Nach Absättigen und Waschen wurden die Vertiefungen mit einem gereinigten Fusionsprotein aus bakterieller Alkalischer Phosphatase (PhoA) und Strep-tag II in den aus dem Graph ersichtlichen Konzentrationen inkubiert. Nach Waschen zur Entfernung von ungebundenem Protein wurde die Aktivität des gebundenen PhoA-Strep-tagII-Fusionsproteins in Gegenwart von p-Nitrophenylphosphat gemessen. Die Daten wurden durch nicht-lineare Regression nach der Methode der kleinsten Fehlerquadrate angepaßt. Die folgenden K_{d}-Werte wurden erhalten: 0,21 µM für Mutein "1"; 0,30 µM für Mutein "2"; 18 µM für rekombinantes wt-Streptavidin.

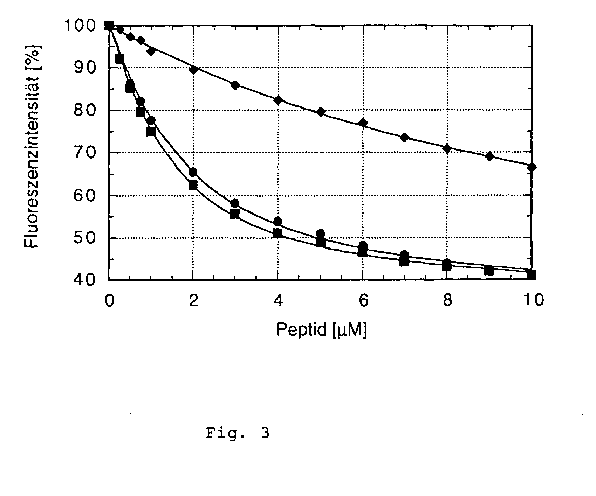

Figur 3 ist eine Graphik, welche die Bindungsaffinität von rekombinantem wt-Streptavidin im Vergleich zu erfindungsgemäßen Streptavidinmuteinen in einer Fluoreszenztitration zeigt.

Eine Lösung des wt-Streptavidins (Raute), des Muteins "1" (Kreis) oder "2" (Quadrat) wurde mit einer Lösung des synthetisierten Strep-tag II-Peptids, welches N-terminal mit Anthranilsäure derivatisiert war, titriert und die Fluoreszenz der Tryptophan- und Tyrosinreste dabei gemessen (Anregung bei 280 nm; Emission bei 340 nm). Die experimentellen Bedingungen sind in Beispiel 6 beschrieben. Durch nicht-lineare Regression der Datenpunkte nach der Theorie einfacher Komplexbildung wurde für den Peptidkomplex ein K_{D}-Wert von 13,0 ± 1,3 µM für wt-Streptavidin ermittelt, während die Mutanten "1" und "2" K_{D}-Werte von 1,37 ± 0,08 µM bzw. 1,02 ± 0,04 µM zeigten.

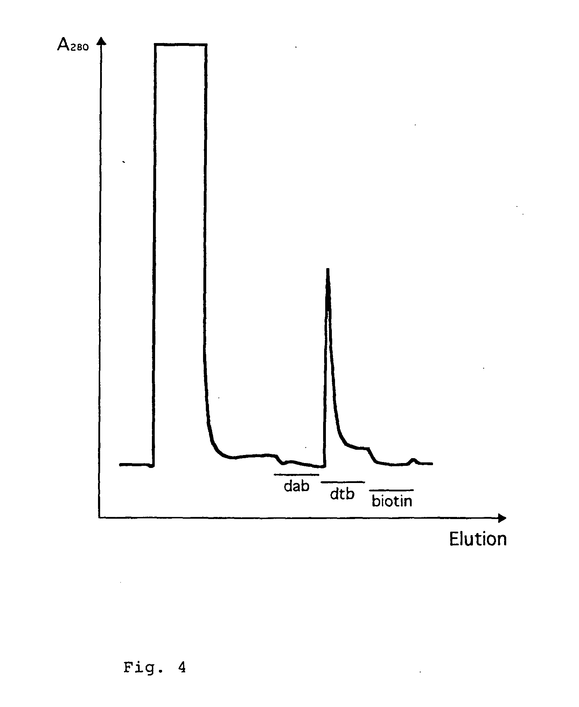

Figur 4 zeigt die Reinigung des Fusionsproteins aus der bakteriellen Alkalischen Phosphatase und Strep-tag II durch Affinitätschromatographie unter Verwendung des immobilisierten erfindungsgemäßen Streptavidinmuteins "1".

Figur 4 zeigt das Elutionsprofil (anhand der Absorption des Eluats bei 280 nm) bei der affinitätschromatographischen Reinigung des Fusionsproteins der bakteriellen Alkalischen Phosphatase mit dem Strep-tag II aus dem bakteriellen periplasmatischen Zellextrakt unter Verwendung des immobilisierten Streptavidinmuteins "1". Die experimentellen Bedingungen sind in Beispiel 4 beschrieben. Nach Entfernung der Wirtsproteine durch Waschen mit Chromatographiepuffer wurde nacheinander mit Lösungen von Diaminobiotin (dab), Desthiobiotin (dtb) und Biotin eluiert. In Gegenwart von Destiobiotin wurde das gebundene Fusionsprotein nahezu quantitativ eluiert. Anschließende Analyse durch SDS-Polyacrylamid-Gelelektrophorese zeigte eine praktisch vollständige Reinheit des so isolierten Proteins.

Weiterhin wird die Erfindung durch das nachfolgende Sequenzprotokoll erläutert. Es zeigen:

| | |
|---|---|
| SEQ ID NO. 1: | die Aminosäuresequenz des Peptidliganden Streptag, |
| SEQ ID NO. 2: | die Aminosäuresequenz des Peptidliganden Streptag II, |
| SEQ ID NO. 3/4: | die Nucleotid- und Aminosäuresequenz von wt-Streptavidin im Bereich der Aminosäuren 44-47, |
| SEQ ID NO. 5/6: | die Nukleotid- und Aminosäuresequenz des Streptavidin-Muteins 1 im Bereich der Aminosäuren 44-47, |
| SEQ ID NO. 7/8: | die Nukleotid- und Aminosäuresequenz des Streptavidin-Muteins 2 im Bereich der Aminosäuren 44-47, |
| SEQ ID NO. 9: | die Nukleotidsequenz des Oligonukleotidprimers P1, |
| SEQ ID NO. 10: | die Nukleotidsequenz des Oligonukleotidprimers P2, |
| SEQ ID NO. 11: | die Nukleotidsequenz des Oligonukleotidprimers P3, |
| SEQ ID NO. 12: | die Nukleotidsequenz des Oligonukleotidprimers P4, |
| SEQ ID NO. 13: | die Nukleotidsequenz des Oligonukleotidprimers P5, |
| SEQ ID NO. 14: | die Nukleotidsequenz des Oligonukleotidprimers P6 und |
| SEQ ID NO. 15: | die Nukleotidsequenz des Oligonukleotidprimers P7. |

### Beispiele

### Allgemeine Methoden

DNA-Manipulationen wurden nach gängigen gentechnischen Methoden durchgeführt (s. z.B. Sambrook et al., Molecular Cloning. A Laboratory Manual (1989), Cold Spring Harbor Press). Zur Klonierung und Expression wurde im allgemeinen der E. coli K12-Stamm JM83 (Yanisch-Peron et al., (1985), Gene 33, 103-119) verwendet, mit Ausnahme der Expression unter Kontrolle des T7-Promotors, die gemäß Schmidt und Skerra (1994), supra, durchgeführt wurde. Sequenzierungen wurden durch Plasmidsequenzierung nach der üblichen Didesoxytechnik durchgeführt, unter Verwendung des T7-Sequenzierkits von Pharmacia, Freiburg. Die Synthese der Primer bzw. Oligonukleotide erfolgte unter Verwendung eines Applied Biosystems DNA-Syntheseautomaten.

### Beispiel 1

### Herstellung einer Expressionsbank für Streptavidinmuteine

Zur Konstruktion des Vektors pASK75-SAp, der die für ein Minimal-Streptavidin kodierende Gensequenz, fusioniert an die kodierende Sequenz des OmpA-Signalpeptids, trägt (vgl. Figur 1), wurde die für Minimal-Streptavidin kodierende Sequenz aus dem Expressionsvektor pSA1 (Schmidt und Skerra, (1994), supra) unter Verwendung der Primer P1 und P2:
P1: 5'-GAG ATA CAG CTG CAG AAG CAG GTA TCA CCG GCA C (SEQ ID NO. 9) und
P2: 5'-CGG ATC AAG CTT ATT AGG AGG CGG CGG ACG GCT TCA C (SEQ ID NO. 10)
und Taq-DNA-Polymerase durch PCR amplifiziert, das Reaktionsprodukt über Gelelektrophorese gereinigt, mit PvuII und HindIII geschnitten und in das mit StuI und HindIII geschnittene Vektorfragment von pASK75 ligiert. Die vollständige Nukleotidsequenz von pASK75 ist in DE-A-44 17 598.1 angegeben. Der dadurch erzeugte Vektor pASK75-SAp umfaßt eine DNA-Sequenz, die für das OmpA-Signalpeptid, fusioniert an Minimal-Streptavidin, beginnend mit Ala¹³ kodiert.

Eine Plasmidbank mit DNA-Sequenzen, die für im Bereich der Aminosäurepositionen 44 bis 47 (bezogen auf wt-Streptavidin) mutagenisierte Streptavidinderivate kodieren, wurde hergestellt durch PCR-Amplifizierung von pASK75-SAp unter Verwendung der folgenden Primer P3 und P4:
P3: 5'-TCG TGA CCG CGG GTG CAG ACG GAG CTC TGA CCG GTA CCT ACN N(C/G)N N(G/T)N N(C/G)N N(G/T)G GCA ACG CCG AGA GCC GCT AC (SEQ ID NO. 11) und
P4: 5'-CGG ATC AAG CTT ATT AGG AGG CGG CGG ACG GCT TCA C (SEQ ID NO. 12).

Auf diese Weise wurden DNA-Sequenzen erzeugt, die an jeder der vier Positionen 44 bis 47 32-fach degenerierte Codons für jeweils alle 20 Aminosäuren bzw. ein Stopcodon enthielten. Zusätzlich wurde an der Stelle im Bereich der Codons für die Aminosäuren 41/42 eine KpnI-Restriktionsstelle erzeugt. Die resultierenden PCR-Produkte wurden durch Gelelektrophorese gereinigt, mit SacII und HindIII gespalten und in das entsprechend gespaltene Vektorfragment von pASK75-SAp ligiert.

Mit dem Vektorgemisch wurden E.coli JM83-Zellen unter Verwendung des Calciumchloridverfahrens (Sambrook et al., 1989) transformiert.

### Beispiel 2

### Identifizierung von Streptavidinmuteinen mit erhöhter Bindungsaffinität für Peptidliganden

Zur Identifizierung von Streptavidinmuteinen mit erhöhter Bindungsaffinität für Peptidliganden wurde ein Fusionsprotein aus der Alkalischen Phosphatase von E.coli (PhoA) und dem Strep-tagII-Peptid (WSHPQFEK) hergestellt, welches an ihren C-Terminus angehängt wurde. Hierzu wurde das vollständige phoA-Gen einschließlich der eigenen Signalsequenz und Translationsinitiationsregion aus chromosomaler E.coli K12 W3110-DNA (Bachmann, Bacteriol. Rev. 36 (1972), 525-557) unter Verwendung der Phosphorthioat-Primer P5 und P6:
P5: 5'-TAA TGT TCT AGA ACA TGG AGA AAA TAA AGT GAA ACA AAG GAC (SEQ ID NO. 13) und
P6: 5'-GCT AGG CGG TTT CAG CCC CAG AGC GGC TTT C (SEQ ID NO. 14)
und Pfu-DNA-Polymerase gemäß einer von Skerra (Nucleic Acids Res. 20 (1992), 3551 bis 3554) publizierten Methode durch PCR amplifiziert. Das auf diese Weise erhaltene PCR-Produkt wurde gereinigt und mit dem Restriktionsenzym XbaI geschnitten. Dieses DNA-Fragment wurde dann in mehreren Schritten unter Austausch der Region zwischen XbaI und Eco47III in das Plasmid pASK75-strepII (konstruiert aus pASK75 durch ortsspezifische Mutagenese unter Verwendung des Oligodeoxynukleotids P7 5'-CAC AGG TCA AGC TTA TTA TTT TTC GAA CTG CGG GTG AGA CCA AGC GCT GCC TGC (SEQ ID NO. 15) inseriert, wobei das Expressionsplasmid pASK75-PhoA strep II erhalten wurde.

Die Proteinproduktion erfolgte in 2 1 LB-Medium mit 100 µg/ml Ampicillin, wobei die Genexpression bei A₅₅₀ = 0,5 durch Zugabe von 0,2 µg/ml Anhydrotetracyclin induziert wurde. Die Induktion erfolgte über Nacht bei einer Temperatur von 37°C. Das PhoA/Strep-tag II Fusionsenzym wurde dann aus der periplasmatischen Zellfraktion durch Streptavidin-Affinitätschromatographie unter Verwendung von Diaminobiotin als Elutionsmittel gemäß der Prozedur von Schmidt und Skerra (1994), supra, gereinigt. Aufgrund des Vorhandenseins von Zn(II)- und Mg(II)-Ionen im aktiven Zentrum des Enzyms enthielt der Chromatographiepuffer kein EDTA.

Die in Beispiel 1 erhaltene Plasmidbank wurde auf einer hydrophilen GVWP-Membran (Millipore, Eschborn) ausplattiert, die auf eine Agarplatte mit LB-Medium enthaltend 100 µg/ml Ampicillin gelegt worden war. Die Membran wurde bei 37°C für 7 bis 8 Stunden inkubiert, bis Kolonien sichtbar wurden.

Dann wurde eine zweite Membran vorbereitet, eine Immobilon P-Membran (Millipore, Eschborn), die mit Anti-Streptavidin-Immunglobulin (Sigma, Deisenhofen) in einer Konzentration von 720 µg/ml in PBS (4 mM KH₂PO₄, 16 mM Na₂HPO₄, 115 mM NaCl) ca. sechs Stunden beschichtet und danach in 3 % w/v Rinderserumalbumin (BSA), 0,5 % v/v Tween in PBS ca. 2 Stunden blockiert wurde.

Diese zweite Membran wurde auf eine M9-Minimalagarplatte, die 100 µg/ml Ampicillin und 0,2 µg/ml Anhydrotetracyclin enthielt, gelegt. Anschließend wurde die GVWP-Membran mit den Kolonien auf der Oberseite auf die zweite Membran gelegt und die relativen Positionen der beiden Membranen markiert. Nach Inkubation über Nacht bei Raumtemperatur wurde die obere Membran mit den Kolonien abgenommen und auf einer frischen LB-Ampicillin-Agarplatte bei 4°C gelagert. Die zweite Membran wurde ebenfalls von der Agarplatte abgenommen und dreimal eine Minute unter Schütteln in PBS/Tween (0,1 % v/v Tween in PBS) gewaschen. Danach wurde die Membran mit 10 ml frischer PBS/- Tween-Lösung, enthaltend das gereinigte PhoA/Strep-tagII-Fusionsprotein (ca. 1-2 µg/ml), versetzt. Nach einstündiger Inkubation bei Raumtemperatur wurde jeweils zweimal in PBS/- Tween und PBS-Puffer nachgewaschen. Die Signalentwicklung erfolgte während 1 bis 2 Stunden in Gegenwart von 10 ml AP-Puffer (100 mM Tris, pH 8,8, 100 mM NaCl, 5 mM MgCl₂) unter Zugabe von 30 µl Brom-Chlor-Indolylphosphat (BCIP) (50 mg/ml in Dimethylformamid) und 5 µl Nitroblau-Tetrazolium (NBT) (75 mg/ml in 70 % v/v Dimethylformamid). Den dabei entstandenen Farbpunkten wurden entsprechende Kolonien auf der ersten Membran zugeordnet. Nach Isolierung und Kultivierung dieser Klone wurden zwei Streptavidinmuteine "1" und "2" identifiziert. Die Nucleotid- und Aminosäuresequenzen in der mutagenisierten Region für wt-Streptavidin und für die Muteine waren wie folgt:

| | | | | | |
|---|---|---|---|---|---|
| wt-Streptavidin | GAG | TCG | GCC | GTC | (SEQ ID NO. 3) |
| | Glu⁴⁴ | Ser⁴⁵ | Ala⁴⁶ | Val⁴⁷ | (SEQ ID NO. 4) |
| | | | | | |
| Mutein "1" | GTC | ACG | GCG | CGT | (SEQ ID NO. 5) |
| | Val | Thr | Ala | Arg | (SEQ ID NO. 6) |
| | | | | | |
| Mutein "2" | ATC | GGT | GCG | AGG | (SEQ ID NO. 7) |
| | Ile | Gly | Ala | Arg | (SEQ ID NO. 8) |

### Beispiel 3

### Herstellung der Streptavidinmuteine im präparativen Maßstab

Zur Herstellung der Streptavidinmuteine im präparativen Maßstab wurde das bekannte Expressionssystem für rekombinantes Minimal-Streptavidin (Schmidt und Skerra (1994), supra) verwendet. Dazu wurde aus dem Vektor pSA1, der die kodierende Region von wt-Streptavidin trägt und den T7-Promotor enthält, der Großteil der kodierenden Region unter Verwendung der singulären SacII- und HindIII-Restriktionsstellen entfernt und durch die entsprechenden Bereiche aus den mutierten pASK75-SAp-Plasmiden setzt. wt-Streptavidin und die Streptavidinmuteine wurden danach in Form cytoplasmatischer Einschlußkörper exprimiert, solubilisiert, renaturiert und durch fraktionierte Ammoniumsulfatpräzipitation gereinigt, wie bei Schmidt und Skerra (1994), supra beschrieben. Die Reinheit der Proteine wurde durch SDS-PAGE unter Verwendung des diskontinuierlichen Puffersystems von Fling und Gregerson (Anal. Biochem. 155 (1986), 83-88) überprüft. Die Charakterisierung der gereinigten, gegen Wasser dialysierten Proteine durch Elektrospray-Ionisations-Massenspektrometrie ergab Massen von 13334 für das rekombinante wt-Streptavidin (theoretisch 13331,5), 13371 für Mutein "1" (theoretisch 13372,6) und 13344 für Mutein "2" (theoretisch 13342,5).

### Beispiel 4

### Affinitätschromatographie

Die in Beispiel 3 hergestellten Streptavidinmuteine sowie wt-Streptavidin wurden mit einer Beladung von 5 mg Protein pro ml gequollenem Gel an NHS-aktivierte Sepharose 4B (Pharmacia Freiburg) gekoppelt (Schmidt & Skerra, 1994, Supra). Nach Blockierung der restlichen aktiven Gruppen mit 100 mM Tris/- HCl, pH 8,0 über Nacht wurden 2 ml des Gels in eine Säule mit einem Durchmesser von 7 mm eingebracht. Um das Verhalten der auf diese Weise immobilisierten Streptavidinmuteine bei der Affinitätsreinigung von Strep-tag oder Strep-tag II-tragenden Fusionsproteinen zu untersuchen, wurde der rekombinante Proteaseinhibitor Cystatin (Schmidt & Skerra 1994, supra), der entweder mit dem Strep-tag oder dem Strep-tag II fusioniert war, sowie das vorstehend erwähnte PhoA/Strep tag-II Fusionsprotein verwendet. Die Fusionsproteine wurden in einem Expressionsystem durch Sekretion in den periplasmatischen Raum produziert und die periplasmatische Zellfraktion wurde wie in Schmidt & Skerra (1994), supra beschrieben, präpariert.

Die Chromatographie erfolgte in Gegenwart von 100 mM Tris/HCl, pH 8,0, enthaltend 1 mM EDTA (außer bei PhoA) unter Verwendung einer Flußrate von ca. 20 ml/h und Messung der Eluatabsorption bei 280 nm. Nach Auftragen einer Probe von 10 ml, entsprechend der periplasmatischen Zellfraktion von 1 l E.coli Kulturmedium, wurde die Säule gewaschen, bis die Absorption bei 280 nm die Basislinie erreicht hatte. Danach wurde gebundenes Protein schrittweise eluiert, durch Auftragen von jeweils 10 ml Diaminobiotin, Desthiobiotin und Biotin (alle von Sigma, Deisenhofen) in einer Konzentration von 2,5 mM in Chromatographiepuffer und in der angegebenen Reihenfolge.

Es zeigte sich, daß im Gegensatz zu wt-Streptavidin die Anwendung von Diaminobiotin bei den Streptavidinmuteinen nicht zu einer Elution führte. Bei Verwendung des mit höherer Affinität an Streptavidin bindenden Biotinderivats Desthiobition wurde auch bei den Muteinen eine Elution in einem scharfen Maximum erreicht. Diese war quantitativ, da bei der nachfolgenden Elution mit Biotin keine wesentliche Menge an Fusionsprotein im Eluat nachgewiesen werden konnte (vgl. Fig. 4).

### Beispiel 5

### ELISA

Zur Bestimmung der Bindungsaffinität der Streptavidinmuteine für den Peptidliganden Strep-tagII wurde ein ELISA durchgeführt.

Die Vertiefungen einer 96-Loch Mikrotiterplatte (Becton Dikkinson an Co., Oxnard, CA) wurden mit 100 µl einer Lösung von rekombinantem wt-Streptavidin bzw. der Muteine "1" oder "2" in einer Konzentration von jeweils 100 µg/ml in 50 mM NaHCO₃, pH 9,6 über Nacht beschichtet. Die Vertiefungen wurden danach 2,5 h mit 3 % w/v BSA, 0,5 % v/v Tween in PBS blockiert. Nach dreimaligem Waschen mit PBS/Tween wurden 50 µl des gleichen Puffers zu jeder Vertiefung gegeben. In die erste Vertiefung in jeder Reihe wurden 20 µl einer Lösung aus 20 µl von 4,85 µM gereinigtem und dialysiertem PhoA/Strep tag II Fusionsprotein plus 30 µl PBS/Tween gegeben und gemischt. Für die weiteren Vertiefungen einer Reihe wurde eine Verdünnungsserie erstellt, indem 50 µl (von insgesamt 100 µl) aus der ersten Vertiefung herauspipettiert und mit dem Inhalt (50 µl) der nächsten Vertiefung in derselben Reihe vermischt wurden usw. Auf diese Weise wurden Konzentrationen des Fusionsproteins zwischen 970 nM in der ersten Vertiefung jeder Reihe und 0,19 nM in der zehnten Vertiefung erhalten.

Nach einstündiger Inkubation wurden die Lösungen entfernt und die Vertiefungen jeweils zweimal mit PBS/Tween und mit PBS gewaschen. Danach wurden 100 µl einer Lösung aus 0,5 mg/ml p-Nitrophenylphosphat in 1 mM ZnSO₄, 5 mM MgCl₂, 1 mM Tris/HCl, pH 8,0 in jede Vertiefung pipettiert. Die Aktivität des gebundenen Fusionsproteins wurde unter Verwendung eines SpektraMAX 250 Photometers (Molecular Devices, Sunnyvale, CA) als Absorptionsänderung bei 410 nm pro Zeit gemessen.

Die Auswertung der Daten erfolgte unter der Annahme eines einfachen Bindungsgleichgewichts zwischen Streptavidin (Mutein) Monomeren (P) und PhoA/Strep-tag II Fusionsprotein (L) wodurch die Dissoziationskonstante zu K_{D} = [P] [L] / [P·L] gegeben ist. Unter der Bedingung, daß [P]_{TOT} = [P] + [P·L] und daß [L] sehr viel größer als [P·L] ist, so daß [L]_{TOT} ungefähr gleich [L] ist, gilt für die Menge des gebundenen Fusionsproteins [P·L] = [L]_{TOT} [P]_{TOT}/(K_{D} + [L]_{TOT}). Die graphische Auswertung der erhaltenen Versuchsergebnisse ist in Figur 2 gezeigt.

Aus den Bindungskurven ist ersichtlich, daß die zwei Streptavidinmuteine eine sehr ähnliche Affinität für das Strep-tag II Fusionsprotein aufweisen, die um mehr als eine Größenordnung über der Affinität von wt-Streptavidin liegt.

### Beispiel 6

### Fluoreszenztitration

Zur Bestimmung der Dissoziationskonstante des 1:1-Komplexes aus den Streptavidinmuteinen (als Monomer betrachtet) und dem Peptidliganden wurde eine Fluoreszenztitration mit dem peptidchemisch synthetisierten Strep-tag II durchgeführt.

Das Peptid mit der Sequenz Abz-Trp-Ser-His-Pro-Gln-Phe-Glu-Lys-COOH (Abz steht für o-Aminobenzoesäure, d.h. Anthranilsäure) wurde an der festen Phase schrittweise aus Fmoc-geschützten Aminosäuren nach dem Fachmann bekannten Methoden in der Reihenfolge vom C-Terminus zum N-Terminus synthetisiert, wobei Abz als Boc-geschütztes Derivat im letzten Schitt gekoppelt wurde. Das Peptid wurde anschließend vom Träger abgespalten und von den Schutzgruppen befreit. Nach Reinigung durch HPLC wurde die Molmasse mittels Felddesorptions-Massenspektrometrie bestätigt.

Die Fluoreszenztitration wurde in einer 1·1 cm²-Quarzküvette, welche bei 25°C thermostatisiert war, mit einem LS50-Fluoreszenzspektrophotometer der Firma Perkin Elmer (Langen) durchgeführt. Die Wellenlängen für Anregungen bzw. Emission betrugen 280 nm sowie 340 nm, bei einer jeweiligen Schlitzbreite von 5 nm. 2 ml der Lösung von wt-Streptavidin bzw. der Muteine "1" und "2", welche wie in Beispiel 3 beschrieben hergestellt und gegen 1 mM EDTA, 100 mM Tris/HCl pH 8,0 dialysiert worden waren, wurden mit einer Konzentration von 1 µM (absorptionsphotometrisch bestimmt für das jeweilige Monomer unter Verwendung eines Extinktionskoeffizienten von ε₂₈₀=40455 M⁻¹ cm⁻¹) in der Küvette vorgelegt. Dann wurden Volumina von 1 µl oder 4 µl einer 0,5 mM Lösung des Peptids in dem gleichen Puffer wiederholt zupipettiert (insgesamt 40 µl) und nach Mischen mit einem Rührfisch die Fluoreszenzintensität abgelesen. Die Auswertung der Daten erfolgte wie bei Figur 3 beschrieben.

## Patentansprüche (Patentansprüche für folgende(n) Vertragsstaat(en): DE, IT, FR)

1. Polypeptid, ausgewählt aus Muteinen von Streptavidin,
**dadurch gekennzeichnet,**
**daß** es (a) innerhalb des Bereichs der Aminosäurepositionen 44 bis 53, bezogen auf die Aminosäuresequenz von Wildtyp-Streptavidin, mindestens eine Mutation enthält und (b) eine mindestens um den Faktor 5 höhere Bindungsaffinität für Peptidliganden, umfassend die Aminosäuresequenz Trp-X-His-Pro-Gln-Phe-Y-Z, wobei X eine beliebige Aminosäure darstellt und Y und Z entweder beide Gly oder Y Glu und Z Arg oder Lys bedeuten, aufweist als wt-Streptavidin und
mit der Maßgabe, dass folgende Streptavidin-Muteine ausgenommen sind, die die Aminosäuresequenz von Ala 13 bis Ala 138, bezogen auf die Aminosäuresequenz von Wildtyp-Streptavidin, besitzen und bei denen
1) an Sequenzposition 45 die Aminosäure Arg,
2) an Sequenzposition 25 die Aminosäure Trp und an Sequenzposition 45 die Aminosäure Arg,
3) an Sequenzposition 45 die Aminosäure Trp und an Sequenzposition 110 die Aminosäure Trp,
4) an Sequenzposition 45 die Aminosäure Tyr und an Sequenzposition 110 die Aminosäure Trp,
5) an Sequenzposition 25 die Aminosäure Trp, an Sequenzposition 45 die Aminosäure Trp und an Sequenzposition 110 die Aminosäure Trp,
6) an Sequenzposition 25 die Aminosäure Trp, an Sequenzposition 45 die Aminosäure Tyr und an Sequenzposition 110 die Aminosäure Trp,
7) an Sequenzposition 27 die Aminosäure Arg, an Sequenzposition 45 die Aminosäure Arg und an Sequenzposition 110 die Aminosäure Trp,
8) an Sequenzposition 27 die Aminosäure Arg, an Sequenzposition 45 die Aminosäure Arg und an Sequenzposition 120 die Aminosäure Ala oder
9) an Sequenzposition 27 die Aminosäure Arg und an Sequenzposition 45 die Aminosäure Arg vorhanden ist.

2. Polypeptid nach Anspruch 1,
**dadurch gekennzeichnet,**
**daß** es das Mutein eines Minimal-Streptavidins ist, das N-terminal im Bereich der Aminosäuren 10 bis 16 von Wildtyp-Streptavidin beginnt und C-terminal im Bereich der Aminosäuren 133-142 von Wildtyp-Streptavidin endet.

3. Polypeptid nach Anspruch 1 oder 2,
**dadurch gekennzeichnet,**
**daß** mindestens eine Mutation im Bereich der Aminosäurepositionen 44 bis 47 vorliegt.

4. Polypeptid nach Anspruch 1 bis 3,
**dadurch gekennzeichnet,**
**daß** an Position 44 Glu durch eine hydrophobe aliphatische Aminosäure ersetzt ist, an Position 45 eine beliebige Aminosäure vorhanden ist, an Position 46 eine hydrophobe aliphatische Aminosäure vorhanden ist oder/und an Position 47 Val durch eine basische Aminosäure ersetzt ist.

5. Polypeptid nach Anspruch 4,
**dadurch gekennzeichnet,**
**daß** an Position 46 Ala vorhanden ist.

6. Polypeptid nach Anspruch 4 oder 5,
**dadurch gekennzeichnet,**
**daß** an Position 47 Arg vorhanden ist.

7. Polypeptid nach Anspruch 6,
**dadurch gekennzeichnet,**
**daß** im Bereich der Aminosäurepositionen 44 bis 47 die Sequenz val⁴⁴-Thr⁴⁵-Ala⁴⁶-Arg⁴⁷ vorliegt.

8. Polypeptid nach Anspruch 6,
**dadurch gekennzeichnet,**
**daß** im Bereich der Aminosäurepositionen 44 bis 47 die Sequenz Ile⁴⁴-Gly⁴⁵-Ala⁴⁶-Arg⁴⁷ vorliegt.

9. Polypeptid nach einem der Ansprüche 1 bis 8,
**dadurch gekennzeichnet,**
**daß** die Bindungsaffinität für den Peptidliganden derart ist, daß eine kompetitive Elution durch Streptavidin-Liganden, ausgewählt aus Biotin, Iminobiotin, Liponsäure, Desthiobiotin, Diaminobiotin, HABA oder/und Dimethyl-HABA erfolgen kann.

10. Polypeptid nach einem der Ansprüche 1 bis 9,
**dadurch gekennzeichnet,**
**daß** die Affinitätskonstante des Komplexes aus Polypeptid und Peptidligand, bezogen auf AWRHPQFGG > 2,7·10⁴ M⁻¹, und bezogen auf WSHPQFEK > 1,4 · 10⁴ M⁻¹ ist.

11. Polypeptid nach einem der Ansprüche 1 bis 10,
**dadurch gekennzeichnet,**
**daß** es mindestens eine Markierungsgruppe trägt.

12. Nukleinsäure,
**dadurch gekennzeichnet,**
**daß** sie eine für das Streptavidinmutein nach einem der Ansprüche 1 bis 10 kodierende Sequenz umfaßt.

13. Vektor, umfassend mindestens eine Kopie einer Nukleinsäure nach Anspruch 12 in operativ funktioneller Umgebung.

14. Zelle,
**dadurch gekennzeichnet,**
**daß** sie mit einem Vektor nach Anspruch 13 transformiert oder transfiziert ist.

15. Verfahren zur Herstellung eines Streptavidinmuteins nach einem der Ansprüche 1 bis 10, **gekennzeichnet durch** die folgenden Schritte:
(a) Transformieren einer geeigneten Wirtszelle mit einem Vektor, der eine für das Streptavidinmutein kodierende Nukleinsäure umfaßt,
(b) Kultivieren der Wirtszelle unter Bedingungen, bei denen eine Expression des Streptavidinmuteins erfolgt,
(c) Gewinnen des Polypeptids.

16. Verfahren nach Anspruch 15,
wobei Schritt (c) Lyse der Wirtszellen, Gewinnung des Streptavidinmuteins in Form von Inclusion bodies und Renaturierung des Streptavidinmuteins umfaßt.

17. Verfahren nach Anspruch 15, wobei die Expression in Schritt (b) die Expression eines Signalpeptid-tragenden Streptavidins und dessen Sekretion in das Periplasma der Wirtszelle oder das Kulturmedium umfaßt.

18. Verfahren nach einem der Ansprüche 15 bis 17, wobei die Wirtszelle E. coli ist.

19. Verwendung eines Streptavidinmuteins nach einem der Ansprüche 1 bis 11 in einem Verfahren zur Isolierung, Reinigung oder Bestimmung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, wobei man eine das zu isolierende, zu reinigende oder zu bestimmende Protein enthaltende Flüssigkeit mit dem Streptavidinmutein unter geeigneten Bedingungen, um eine Bindung der Peptidsequenz an das Streptavidinmutein zu bewirken, in Kontakt bringt, den resultierenden Komplex aus der Flüssigkeit abtrennt und das Protein gegebenenfalls aus dem Komplex freisetzt oder bestimmt.

20. Verwendung nach Anspruch 19,
**dadurch gekennzeichnet,**
**daß** das Streptavidinmutein an eine Festphase gebunden oder mit ihr bindefähig ist.

21. Verwendung nach Anspruch 19 oder 20,
**dadurch gekennzeichnet,**
**daß** man zum Freisetzen des Fusionsproteins aus dem Komplex den Komplex mit einer ausreichenden Menge eines Liganden für das Streptavidinmutein ausgewählt aus Biotin und Derivaten davon inkubiert.

22. Verwendung nach Anspruch 21,
**dadurch gekennzeichnet,**
**daß** das Biotinderivat Desthiobiotin ist.

23. Verwendung eines markierten Streptavidinmuteins nach Anspruch 11 in einem Verfahren zur Bestimmung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, wobei man das zu bestimmende Protein mit dem markierten Streptavidinmutein unter geeigneten Bedingungen, um eine Bindung der Peptidsequenz an das Streptavidinmutein zu bewirken, in Kontakt bringt, und die Markierung bestimmt.

24. Verwendung eines Streptavidinmuteins nach einem der Ansprüche 1 bis 11 zur Immobilisierung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, an einer Festphase.

25. Verwendung nach einem der Ansprüche 19 bis 23,
**dadurch gekennzeichnet,**
**daß** die Peptidsequenz ausgewählt wird aus AWRHPQFGG oder WSHPQFEK.

26. Verwendung eines Streptavidinmuteins nach einem der Ansprüche 1 bis 11 zur Bestimmung oder Gewinnung von Substanzen, die eine mit Streptavidin bindefähige Gruppe tragen.

27. Reagenzienkit zur Verwendung gemäß einem der Ansprüche 19 bis 26, umfassend ein Streptavidinmutein nach einem der Ansprüche 1 bis 11 sowie gegebenenfalls übliche Puffer-, Hilfs- und Zusatzstoffe.

## Patentansprüche (Patentansprüche für folgende(n) Vertragsstaat(en): BE, CH, DK, FI, GB, NL SE)

1. Polypeptid, ausgewählt aus Muteinen von Streptavidin,
**dadurch gekennzeichnet, daß** es (a) innerhalb des Bereichs der Aminosäurepositionen 44 bis 53, bezogen auf die Aminosäuresequenz von Wildtyp-Streptavidin, mindestens eine Mutation enthält und (b) eine mindestens um den Faktor 5 höhere Bindungsaffinität für Peptidliganden, umfassend die Aminosäuresequenz Trp-X-His-Pro-Gln-Phe-Y-Z,
wobei X eine beliebige Aminosäure darstellt und Y und Z entweder beide Gly oder Y Glu und Z Arg oder Lys bedeuten, aufweist als wt-Streptavidin.

2. Polypeptid nach Anspruch 1,
**dadurch gekennzeichnet,**
**daß** es das Mutein eines Minimal-Streptavidins ist, das N-terminal im Bereich der Aminosäuren 10 bis 16 von Wildtyp-Streptavidin beginnt und C-terminal im Bereich der Aminosäuren 133-142 von Wildtyp-Streptavidin endet.

3. Polypeptid nach Anspruch 1 oder 2,
**dadurch gekennzeichnet,**
**daß** mindestens eine Mutation im Bereich der Aminosäurepositionen 44 bis 47 vorliegt.

4. Polypeptid nach Anspruch 1 bis 3,
**dadurch gekennzeichnet,**
**daß** an Position 44 Glu durch eine hydrophobe aliphatische Aminosäure ersetzt ist, an Position 45 eine beliebige Aminosäure vorhanden ist, an Position 46 eine hydrophobe aliphatische Aminosäure vorhanden ist oder/und an Position 47 Val durch eine basische Aminosäure ersetzt ist.

5. Polypeptid nach Anspruch 4,
**dadurch gekennzeichnet,**
**daß** an Position 46 Ala vorhanden ist.

6. Polypeptid nach Anspruch 4 oder 5,
**dadurch gekennzeichnet,**
**daß** an Position 47 Arg vorhanden ist.

7. Polypeptid nach Anspruch 6,
**dadurch gekennzeichnet,**
**daß** im Bereich der Aminosäurepositionen 44 bis 47 die Sequenz val⁴⁴-Thr⁴⁵-Ala⁴⁶-Arg⁴⁷ vorliegt.

8. Polypeptid nach Anspruch 6,
**dadurch gekennzeichnet,**
**daß** im Bereich der Aminosäurepositionen 44 bis 47 die Sequenz Ile⁴⁴-Gly⁴⁵-Ala⁴⁶-Arg⁴⁷ vorliegt.

9. Polypeptid nach einem der Ansprüche 1 bis 8,
**dadurch gekennzeichnet,**
**daß** die Bindungsaffinität für den Peptidliganden derart ist, daß eine kompetitive Elution durch Streptavidin-Liganden, ausgewählt aus Biotin, Iminobiotin, Liponsäure, Desthiobiotin, Diaminobiotin, HABA oder/und Dimethyl-HABA erfolgen kann.

10. Polypeptid nach einem der Ansprüche 1 bis 9,
**dadurch gekennzeichnet,**
**daß** die Affinitätskonstante des Komplexes aus Polypeptid und Peptidligand, bezogen auf AWRHPQFGG > 2,7·10⁴ M⁻¹, und bezogen auf WSHPQFEK > 1,4 · 10⁴ M⁻¹ ist.

11. Polypeptid nach einem der Ansprüche 1 bis 10,
**dadurch gekennzeichnet,**
**daß** es mindestens eine Markierungsgruppe trägt.

12. Nukleinsäure,
**dadurch gekennzeichnet,**
**daß** sie eine für das Streptavidinmutein nach einem der Ansprüche 1 bis 10 kodierende Sequenz umfaßt.

13. Vektor, umfassend mindestens eine Kopie einer Nukleinsäure nach Anspruch 12 in operativ funktioneller Umgebung.

14. Zelle,
**dadurch gekennzeichnet,**
**daß** sie mit einem Vektor nach Anspruch 13 transformiert oder transfiziert ist.

15. Verfahren zur Herstellung eines Streptavidinmuteins nach einem der Ansprüche 1 bis 10, **gekennzeichnet durch** die folgenden Schritte:
(a) Transformieren einer geeigneten Wirtszelle mit einem Vektor, der eine für das Streptavidinmutein kodierende Nukleinsäure umfaßt,
(b) Kultivieren der Wirtszelle unter Bedingungen, bei denen eine Expression des Streptavidinmuteins erfolgt,
(c) Gewinnen des Polypeptids.

16. Verfahren nach Anspruch 15,
wobei Schritt (c) Lyse der Wirtszellen, Gewinnung des Streptavidinmuteins in Form von Inclusion bodies und Renaturierung des Streptavidinmuteins umfaßt.

17. Verfahren nach Anspruch 15, wobei die Expression in Schritt (b) die Expression eines Signalpeptid-tragenden Streptavidins und dessen Sekretion in das Periplasma der Wirtszelle oder das Kulturmedium umfaßt.

18. Verfahren nach einem der Ansprüche 15 bis 17, wobei die Wirtszelle E. coli ist.

19. Verwendung eines Streptavidinmuteins nach einem der Ansprüche 1 bis 11 in einem Verfahren zur Isolierung, Reinigung oder Bestimmung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, wobei man eine das zu isolierende, zu reinigende oder zu bestimmende Protein enthaltende Flüssigkeit mit dem Streptavidinmutein unter geeigneten Bedingungen, um eine Bindung der Peptidsequenz an das Streptavidinmutein zu bewirken, in Kontakt bringt, den resultierenden Komplex aus der Flüssigkeit abtrennt und das Protein gegebenenfalls aus dem Komplex freisetzt oder bestimmt.

20. Verwendung nach Anspruch 19,
**dadurch gekennzeichnet,**
**daß** das Streptavidinmutein an eine Festphase gebunden oder mit ihr bindefähig ist.

21. Verwendung nach Anspruch 19 oder 20,
**dadurch gekennzeichnet,**
**daß** man zum Freisetzen des Fusionsproteins aus dem Komplex den Komplex mit einer ausreichenden Menge eines Liganden für das Streptavidinmutein ausgewählt aus Biotin und Derivaten davon inkubiert.

22. Verwendung nach Anspruch 21,
**dadurch gekennzeichnet,**
**daß** das Biotinderivat Desthiobiotin ist.

23. Verwendung eines markierten Streptavidinmuteins nach Anspruch 11 in einem Verfahren zur Bestimmung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, wobei man das zu bestimmende Protein mit dem markierten Streptavidinmutein unter geeigneten Bedingungen, um eine Bindung der Peptidsequenz an das Streptavidinmutein zu bewirken, in Kontakt bringt, und die Markierung bestimmt.

24. Verwendung eines Streptavidinmuteins nach einem der Ansprüche 1 bis 11 zur Immobilisierung eines Proteins, das mit einer Peptidsequenz der Formel Trp-X-His-Pro-Gln-Phe-Y-Z, wobei X eine beliebige Aminosäure darstellt und Y und Z entweder beide Gly oder Y Glu und Z Arg oder Lys bedeuten, fusioniert ist, an einer Festphase.

25. Verwendung nach einem der Ansprüche 19 bis 23,
**dadurch gekennzeichnet,**
**daß** die Peptidsequenz ausgewählt wird aus AWRHPQFGG oder WSHPQFEK.

26. Verwendung eines Streptavidinmuteins nach einem der Ansprüche 1 bis 11 zur Bestimmung oder Gewinnung von Substanzen, die eine mit Streptavidin bindefähige Gruppe tragen.

27. Reagenzienkit zur Verwendung gemäß einem der Ansprüche 19 bis 26, umfassend ein Streptavidinmutein nach einem der Ansprüche 1 bis 11 sowie gegebenenfalls übliche Puffer-, Hilfs- und Zusatzstoffe.

## Claims (Claims for the following Contracting State(s): DE, IT, FR)

1. Polypeptide, selected from muteins of streptavidin,
**characterized in that** it (a) contains at least one mutation in the region of the amino acid positions 44 to 53 with reference to the amino acid sequence of wild type streptavidin and (b) has a binding affinity that is increased compared to wild type-streptavidin by at least a factor of 5 for peptide ligands comprising the amino acid sequence Trp-X-His-Pro-Gln-Phe-Y-Z, in which X represents any amino acid and Y and Z either both denote Gly or Y denotes Glu and Z denotes Arg or Lys,
with the proviso that the following streptavidin muteins are excluded, which have the amino acid sequence of Ala 13 to Ala 138 with reference to the amino acid sequence of wild type streptavidin and in which
1) at sequence position 45 the amino acid Arg,
2) at sequence position 25 the amino acid Trp and at sequence position 45 the amino acid Arg,
3) at sequence position 45 the amino acid Trp and at sequence position 110 the amino acid Trp,
4) at sequence position 45 the amino acid Tyr and at sequence position 110 the amino acid Trp,
5) at sequence position 25 the amino acid Trp, at sequence position 45 the amino acid Trp and at sequence position 110 the amino acid Trp,
6) at sequence position 25 the amino acid Trp, at sequence position 45 the amino acid Tyr and at sequence position 110 the amino acid Trp,
7) at sequence position 27 the amino acid Arg, at sequence position 45 the amino acid Arg and at sequence position 110 the amino acid Trp,
8) at sequence position 27 the amino acid Arg, at sequence position 45 the amino acid Arg and at sequence position 120 the amino acid Ala or 9) at sequence position 27 the amino acid Arg und at sequence position 45 the amino acid Arg is present.

2. Polypeptide according to claim 1,
**characterized in that**
it is the mutein of a minimal streptavidin which begins N-terminally in the region of the amino acids 10 to 16 of wild type streptavidin and terminates C-terminally in the region of the amino acids 133-142 of wild type streptavidin.

3. Polypeptide according to claim 1 or 2,
**characterized in that**
at least one mutation is present in the region of amino acid positions 44 to 47.

4. Polypeptide according to any of claims 1 to 3,
**characterized in that**
Glu is replaced by a hydrophobic aliphatic amino acid at position 44, any amino acid is present at position 45, a hydrophobic aliphatic amino acid is present at position 46 or/and Val is replaced by a basic amino acid at position 47.

5. Polypeptide according to claim 4, **characterized in that**
Ala is present at position 46.

6. Polypeptide according to claim 4 or 5, **characterized in that** Arg is present at position 47.

7. Polypeptide according to claim 6,
**characterized in that**
the sequence Val⁴⁴ -Thr⁴⁵ -Ala⁴⁶ -Arg⁴⁷ is present in the region of amino acid positions 44 to 47.

8. Polypeptide according to claim 6,
**characterized in that**
the sequence Ile⁴⁴ -Gly⁴⁵ -Ala⁴⁶ -Arg⁴⁷ is present in the region of amino acid positions 44 to 47.

9. Polypeptide according to any one of claims 1 to 8,
**characterized in that**
the binding affinity for the peptide ligand is such that a competitive elution can take place by streptavidin ligands selected from biotin, iminobiotin, lipoic acid, desthiobiotin, diaminobiotin, HABA or/and dimethyl-HABA.

10. Polypeptide according to any of claims 1 to 9,
**characterized in that**
the affinity constant of the complex of polypeptide and peptide ligand is >2.7 · 10⁴ M⁻¹ with respect to AWRHPQFGG, and >1.4 ·10⁴ M⁻¹ with respect to WSHPQFEK.

11. Polypeptide as claimed in one of claims 1 to 10,
**characterized in that**
it carries at least one labeling group.

12. Nucleic acid,
**characterized in that**
it comprises a sequence coding for the streptavidin mutein according to any one of claims 1 to 10.

13. Vector comprising at least one copy of a nucleic acid according to claim 12 in an operatively functionally environment.

14. Cell,
**characterized in that**
it is transformed or transfected with a vector as claimed in claim 13.

15. Process for the production of a streptavidin mutein according to any of claims 1 to 10, **characterized by** the following steps:
(a) transforming a suitable host cell with a vector which contains a nucleic acid coding for the streptavidin mutein,
(b) culturing the host cell under conditions in which an expression of the streptavidin mutein takes place,
(c) isolating the polypeptide.

16. Method according to claim 15,
wherein step (c) comprises lysing the host cells, isolating the streptavidin mutein in the form of inclusion bodies and renaturing the streptavidin mutein.

17. Method according to claim 15,
wherein the expression in step (b) comprises the expression of a signal peptide-carrying streptavidin and its secretion into the periplasma of the host cell or the culture medium.

18. Method according to any one of claims 15 to 17, wherein the host cell is E. coli.

19. Use of a streptavidin mutein according to any one of claims 1 to 11 in a method for the isolation, purification or determination of a protein that is fused with a peptide sequence of the formula Trp-X-His-Pro-Gln-Phe-Y-Z in which X represents any amino acid and Y and Z either both denote Gly or Y denotes Glu and Z denotes Arg or Lys, comprising contacting a liquid containing the protein that is to be isolated, to be purified or to be determined with the streptavidin mutein under conditions suitable for causing binding of the peptide sequence to the streptavidin mutein, separating the resulting complex from said liquid, and optionally releasing the protein from the complex or determining the protein.

20. Use according to claim 19,
**characterized in that**
the streptavidin mutein is bound to a solid phase or is capable of binding thereto.

21. Use according to claim 19 or 20,
**characterized in that**
in order to release the fusion protein from the complex the complex is incubated with an adequate amount of a ligand for the streptavidin mutein selected from biotin and derivatives thereof.

22. Use according to claim 21,
**characterized in that**
the biotin derivative is desthiobiotin.

23. Use of a labeled streptavidin mutein according to claim 11 in a method for determination of a protein that is fused with a peptide sequence of the formula Trp-X-His-Pro-Gln-Phe-Y-Z in which X represents any amino acid and Y and Z either both denote Gly or Y denotes Glu and Z denotes Arg or Lys, comprising contacting the protein that is to be determined with the labeled streptavidin mutein under suitable conditions in order to cause binding of the peptide sequence to the streptavidin mutein and determining the label.

24. Use of a streptavidin mutein according to any one of claims 1 to 11 for the immobilization to a solid phase of a protein that is fused with a peptide sequence of the formula Trp-X-His-Pro-Gln-Phe-Y-Z in which X represents any amino acid and Y and Z either both denote Gly or Y denotes Glu and Z denotes Arg or Lys.

25. Use according to any of claims 19 to 23,
**characterized in that**
the peptide sequence is selected from AWRHPQFGG or WSHPQFEK.

26. Use of streptavidin mutein according to any one of claims 1 to 11 for determination or isolation of substances which carry a group capable of binding to streptavidin.

27. Reagent kit for use according to any one of claims 19 to 26 comprising a streptavidin mutein according to any one of claims 1 to 11, and optionally conventional buffer reagents, auxiliary substance, or additives.

## Claims (Claims for the following Contracting State(s): BE, CH, DK, FI, GB, NL SE)

1. Polypeptide, selected from muteins of streptavidin,
**characterized in that** it (a) contains at least one mutation in the region of the amino acid positions 44 to 53 with reference to the amino acid sequence of wild type streptavidin and (b) has a binding affinity that is increased compared to wild type-streptavidin by at least a factor of 5 for peptide ligands comprising the amino acid sequence Trp-X-His-Pro-Gln-Phe-Y-Z, in which X represents any amino acid and Y and Z either both denote Gly or Y denotes Glu and Z denotes Arg or Lys.

2. Polypeptide according to claim 1,
**characterized in that**
it is the mutein of a minimal streptavidin which begins N-terminally in the region of the amino acids 10 to 16 of wild type streptavidin and terminates C-terminally in the region of the amino acids 133-142 of wild type streptavidin.

3. Polypeptide according to claim 1 or 2,
**characterized in that**
at least one mutation is present in the region of amino acid positions 44 to 47.

4. Polypeptide according to any of claims 1 to 3,
**characterized in that**
Glu is replaced by a hydrophobic aliphatic amino acid at position 44, any amino acid is present at position 45, a hydrophobic aliphatic amino acid is present at position 46 or/and Val is replaced by a basic amino acid at position 47.

5. Polypeptide according to claim 4, **characterized in that**
Ala is present at position 46.

6. Polypeptide according to claim 4 or 5, **characterized in that** Arg is present at position 47.

7. Polypeptide according to claim 6,
**characterized in that**
the sequence Val⁴⁴ -Thr⁴⁵ -Ala⁴⁶ -Arg⁴⁷ is present in the region of amino acid positions 44 to 47.

8. Polypeptide according to claim 6,
**characterized in that**
the sequence Ile⁴⁴ -Gly⁴⁵ -Ala⁴⁶ -Arg⁴⁷ is present in the region of amino acid positions 44 to 47.

9. Polypeptide according to any one of claims 1 to 8,
**characterized in that**
the binding affinity for the peptide ligand is such that a competitive elution can take place by streptavidin ligands selected from biotin, iminobiotin, lipoic acid, desthiobiotin, diaminobiotin, HABA or/and dimethyl-HABA.

10. Polypeptide according to any of claims 1 to 9,
**characterized in that**
the affinity constant of the complex of polypeptide and peptide ligand is >2.7 · 10⁴ M⁻¹ with respect to AWRHPQFGG, and >1.4 ·10⁴ M⁻¹ with respect to WSHPQFEK.

11. Polypeptide as claimed in one of claims 1 to 10,
**characterized in that**
it carries at least one labeling group.

12. Nucleic acid,
**characterized in that**
it comprises a sequence coding for the streptavidin mutein according to any one of claims 1 to 10.

13. Vector comprising at least one copy of a nucleic acid according to claim 12 in an operatively functionally environment.

14. Cell,
**characterized in that**
it is transformed or transfected with a vector as claimed in claim 13.

15. Process for the production of a streptavidin mutein according to any of claims 1 to 10, **characterized by** the following steps:
(a) transforming a suitable host cell with a vector which contains a nucleic acid coding for the streptavidin mutein,
(b) culturing the host cell under conditions in which an expression of the streptavidin mutein takes place,
(c) isolating the polypeptide.

16. Method according to claim 15,
wherein step (c) comprises lysing the host cells, isolating the streptavidin mutein in the form of inclusion bodies and renaturing the streptavidin mutein.

17. Method according to claim 15,
wherein the expression in step (b) comprises the expression of a signal peptide-carrying streptavidin and its secretion into the periplasma of the host cell or the culture medium.

18. Method according to any one of claims 15 to 17, wherein the host cell is E. coli.

19. Use of a streptavidin mutein according to any one of claims 1 to 11 in a method for the isolation, purification or determination of a protein that is fused with a peptide sequence of the formula Trp-X-His-Pro-Gln-Phe-Y-Z in which X represents any amino acid and Y and Z either both denote Gly or Y denotes Glu and Z denotes Arg or Lys, comprising contacting a liquid containing the protein that is to be isolated, to be purified or to be determined with the streptavidin mutein under conditions suitable for causing binding of the peptide sequence to the streptavidin mutein, separating the resulting complex from said liquid, and optionally releasing the protein from the complex or determining the protein.

20. Use according to claim 19,
**characterized in that**
the streptavidin mutein is bound to a solid phase or is capable of binding thereto.

21. Use according to claim 19 or 20,
**characterized in that**
in order to release the fusion protein from the complex the complex is incubated with an adequate amount of a ligand for the streptavidin mutein selected from biotin and derivatives thereof.

22. Use according to claim 21,
**characterized in that**
the biotin derivative is desthiobiotin.

23. Use of a labeled streptavidin mutein according to claim 11 in a method for determination of a protein that is fused with a peptide sequence of the formula Trp-X-His-Pro-Gln-Phe-Y-Z in which X represents any amino acid and Y and Z either both denote Gly or Y denotes Glu and Z denotes Arg or Lys, comprising contacting the protein that is to be determined with the labeled streptavidin mutein under suitable conditions in order to cause binding of the peptide sequence to the streptavidin mutein and determining the label.

24. Use of a streptavidin mutein according to any one of claims 1 to 11 for the immobilization to a solid phase of a protein that is fused with a peptide sequence of the formula Trp-X-His-Pro-Gln-Phe-Y-Z in which X represents any amino acid and Y and Z either both denote Gly or Y denotes Glu and Z denotes Arg or Lys.

25. Use according to any of claims 19 to 23,
**characterized in that**
the peptide sequence is selected from AWRHPQFGG or WSHPQFEK.

26. Use of streptavidin mutein according to any one of claims 1 to 11 for determination or isolation of substances which carry a group capable of binding to streptavidin.

27. Reagent kit for use according to any one of claims 19 to 26 comprising a streptavidin mutein according to any one of claims 1 to 11, and optionally conventional buffer reagents, auxiliary substance, or additives.

## Revendications (Revendications pour l'(les) Etat(s) contractant(s) suivant(s): DE, IT, FR)

1. Polypeptide choisi parmi des mutéines de la streptavidine,
**caractérisé en ce que**
(a) il contient au moins une mutation dans la zone des positions d'acides aminés 44 à 53, par rapport à la séquence d'acides aminés de la streptavidine de type sauvage, et (b) il présente une affinité de liaison supérieure d'au moins un facteur 5 pour des ligands peptidiques, comprenant la séquence d'acides aminés Trp-X-His-Pro-Gln-Phe-Y-Z, où X représente n'importe quel acide aminé et Y et Z représentent tous deux un résidu Gly ou Y représente un résidu Glu et Z un résidu Arg ou Lys, à celle de la streptavidine de type sauvage et
à condition d'exclure les mutéines de streptavidine suivantes, qui possèdent la séquence d'acides aminés s'étendant du résidu Ala 13 au résidu Ala 138, par rapport à la séquence d'acides aminés de la streptavidine de type sauvage, et dans lesquelles on trouve
1) l'acide aminé Arg en position de séquence 45,
2) l'acide aminé Trp en position de séquence 25 et l'acide aminé Arg en position de séquence 45,
3) l'acide aminé Trp en position de séquence 45 et l'acide aminé Trp en position de séquence 110,
4) l'acide aminé Tyr en position de séquence 45 et l'acide aminé Trp en position de séquence 110,
5) l'acide aminé Trp en position de séquence 25, l'acide aminé Trp en position de séquence 45 et l'acide aminé Trp en position de séquence 110,
6) l'acide aminé Trp en position de séquence 25, l'acide aminé Tyr en position de séquence 45 et l'acide aminé Trp en position de séquence 110,
7) l'acide aminé Arg en position de séquence 27, l'acide aminé Arg en position de séquence 45 et l'acide aminé Trp en position de séquence 110,
8) l'acide aminé Arg en position de séquence 27, l'acide aminé Arg en position de séquence 45 et l'acide aminé Ala en position de séquence 120, ou
9) l'acide aminé Arg en position de séquence 27 et l'acide aminé Arg en position de séquence 45.

2. Polypeptide selon la revendication 1,
**caractérisé en ce que**
il s'agit de la mutéine d'une streptavidine minimale qui débute à l'extrémité N-terminale dans la zone des acides aminés 10 à 16 de la streptavidine de type sauvage et se termine à l'extrémité C-terminale dans la zone des acides aminés 133 à 142 de la streptavidine de type sauvage.

3. Polypeptide selon la revendication 1 ou 2,
**caractérisé en ce que**
il y a au moins une mutation dans la zone des positions d'acides aminés 44 à 47.

4. Polypeptide selon les revendications 1 à 3,
**caractérisé en ce que**
un résidu Glu en position 44 est remplacé par un acide aminé aliphatique hydrophobe, un acide aminé quelconque est présent en position 45, un acide aminé aliphatique hydrophobe est présent en position 46 et/ou un résidu Val en position 47 est remplacé par un acide aminé basique.

5. Polypeptide selon la revendication 4,
**caractérisé en ce que**
un résidu Ala est présent en position 46.

6. Polypeptide selon la revendication 4 ou 5,
**caractérisé en ce que**
un résidu Arg est présent en position 47.

7. Polypeptide selon la revendication 6,
**caractérisé en ce que**
il y a la séquence Val⁴⁴-Thr⁴⁵-Ala⁴⁶-Arg⁴⁷ dans la zone des positions d'acides aminés 44 à 47.

8. Polypeptide selon la revendication 6,
**caractérisé en ce que**
il y a la séquence Ile⁴⁴-Gly⁴⁵-Ala⁴⁶-Arg⁴⁷ dans la zone des positions d'acides aminés 44 à 47.

9. Polypeptide selon l'une quelconque des revendications 1 à 8, **caractérisé en ce que**
l'affinité de liaison pour les ligands peptidiques est telle qu'une élution de compétition avec des ligands de streptavidine, choisis parmi la biotine, l'iminobiotine, l'acide lipoïque, la desthiobiotine, la diaminobiotine, le HABA et/ou le diméthyl-HABA puisse se faire.

10. Polypeptide selon l'une quelconque des revendications 1 à 9, **caractérisé en ce que**
la constante d'affinité du complexe constitué d'un polypeptide et d'un ligand peptidique, par rapport à la séquence AWRHPQFGG, est > 2,7·10⁴ M⁻¹ et, par rapport à la séquence WSHPQFEK, > 1,4·10⁴ M⁻¹.

11. Polypeptide selon l'une quelconque des revendications 1 à 10, **caractérisé en ce que**
il porte au moins un groupement de marquage.

12. Acide nucléique,
**caractérisé en ce que**
il comprend une séquence codant pour la mutéine de la streptavidine selon l'une quelconque des revendications 1 à 10.

13. Vecteur, comprenant au moins une copie d'un acide nucléique selon la revendication 12 dans un environnement fonctionnel au plan opérationnel.

14. Cellule,
**caractérisée en ce que**
elle fait l'objet d'une transformation ou d'une transfection avec un vecteur selon la revendication 13.

15. Procédé de fabrication d'une mutéine de la streptavidine selon l'une quelconque des revendications 1 à 10, **caractérisé par** les étapes suivantes :
(a) transformation d'une cellule hôte appropriée avec un vecteur, qui comprend un acide nucléique codant pour la mutéine de la streptavidine,
(b) mise en culture de la cellule hôte dans des conditions permettant une expression de la mutéine de la streptavidine, et
(c) obtention du polypeptide.

16. Procédé selon la revendication 15,
dans lequel l'étape (c) comprend la lyse des cellules hôtes, l'obtention de la mutéine de la streptavidine sous la forme de corps d'inclusion et la renaturation de la mutéine de la streptavidine.

17. Procédé selon la revendication 15, dans lequel l'expression réalisée à l'étape (b) comprend l'expression d'une streptavidine portant un peptide signal et sa sécrétion dans le périplasme de la cellule hôte ou dans le milieu de culture.

18. Procédé selon l'une quelconque des revendications 15 à 17, dans lequel la cellule hôte est E. coli.

19. Utilisation d'une mutéine de la streptavidine selon l'une quelconque des revendications 1 à 11 dans un procédé pour isoler, purifier ou déterminer une protéine, qui est fusionnée avec une séquence peptidique de formule Trp-X-His-Pro-Gln-Phe-Y-Z, où X représente n'importe quel acide aminé et Y et Z représentent tous deux le résidu Gly ou Y représente le résidu Glu et Z représente le résidu Arg ou Lys, dans laquelle on met en contact un liquide contenant la protéine à isoler, à purifier ou à déterminer avec la mutéine de la streptavidine dans des conditions appropriées pour assurer une liaison de la séquence peptidique à la mutéine de la streptavidine, on sépare le complexe obtenu du liquide et on libère ou on détermine la protéine éventuellement à partir du complexe.

20. Utilisation selon la revendication 19,
**caractérisée en ce que**
la mutéine de la streptavidine est liée à une phase solide ou est capable de s'y lier.

21. Utilisation selon la revendication 19 ou 20,
**caractérisée en ce que,**
pour libérer la protéine de fusion du complexe, on incube le complexe avec une quantité suffisante d'un ligand pour la mutéine de la streptavidine, qui est choisi parmi la biotine et ses dérivés.

22. Utilisation selon la revendication 21,
**caractérisée en ce que**
le dérivé de biotine est la desthiobiotine.

23. Utilisation d'une mutéine de la streptavidine marquée selon la revendication 11 dans un procédé de détermination d'une protéine, qui est fusionnée avec une séquence peptidique de formule Trp-X-His-Pro-Gln-Phe-Y-Z, où X représente n'importe quel acide aminé et Y et Z représentent tous deux un résidu Gly ou Y représente un résidu Glu et Z représente un résidu Arg ou Lys, dans laquelle on met en contact la protéine à déterminer avec la mutéine de la streptavidine marquée dans des conditions appropriées pour assurer une liaison de la séquence peptidique à la mutéine de la streptavidine et on détermine le marquage.

24. Utilisation d'une mutéine de la streptavidine selon l'une quelconque des revendications 1 à 11 pour immobiliser une protéine, qui est fusionnée avec une séquence peptidique de formule Trp-X-His-Pro-Gln-Phe-Y-Z, où X représente n'importe quel acide aminé et Y et Z représentent tous deux un résidu Gly ou Y représente un résidu Glu et Z représente un résidu Arg ou Lys, sur une phase solide.

25. Utilisation selon l'une quelconque des revendications 19 à 23, **caractérisée en ce que**
l'on choisit la séquence peptidique parmi les séquences suivantes : AWRHPQFGG ou WSHPQFEK.

26. Utilisation d'une mutéine de la streptavidine selon l'une quelconque des revendications 1 à 11 pour déterminer ou obtenir des substances qui portent un groupement susceptible de se lier à la streptavidine.

27. Trousse de réactifs pour l'utilisation selon l'une quelconque des revendications 19 à 26, comprenant une mutéine de la streptavidine selon l'une quelconque des revendications 1 à 11, ainsi qu'éventuellement des substances tampons, des adjuvants et des additifs.

## Revendications (Revendications pour l'(les) Etat(s) contractant(s) suivant(s): BE, CH, DK, FI, GB, NL SE)

1. Polypeptide choisi parmi des mutéines de la streptavidine,
**caractérisé en ce que**
(a) il contient au moins une mutation dans la zone des positions d'acides aminés 44 à 53, par rapport à la séquence d'acides aminés de la streptavidine de type sauvage, et (b) il présente une affinité de liaison supérieure d'au moins un facteur 5 pour des ligands peptidiques, comprenant la séquence d'acides aminés Trp-X-His-Pro-Gln-Phe-Y-Z, où X représente n'importe quel acide aminé et Y et Z représentent tous deux un résidu Gly ou Y représente un résidu Glu et Z un résidu Arg ou Lys, à celle de la streptavidine de type sauvage.

2. Polypeptide selon la revendication 1,
**caractérisé en ce que**
il s'agit de la mutéine d'une streptavidine minimale qui débute à l'extrémité N-terminale dans la zone des acides aminés 10 à 16 de la streptavidine de type sauvage et se termine à l'extrémité C-terminale dans la zone des acides aminés 133 à 142 de la streptavidine de type sauvage.

3. Polypeptide selon la revendication 1 ou 2,
**caractérisé en ce que**
il y a au moins une mutation dans la zone des positions d'acides aminés 44 à 47.

4. Polypeptide selon les revendications 1 à 3,
**caractérisé en ce que**
un résidu Glu en position 44 est remplacé par un acide aminé aliphatique hydrophobe, un acide aminé quelconque est présent en position 45, un acide aminé aliphatique hydrophobe est présent en position 46 et/ou un résidu Val en position 47 est remplacé par un acide aminé basique.

5. Polypeptide selon la revendication 4,
**caractérisé en ce que**
un résidu Ala est présent en position 46.

6. Polypeptide selon la revendication 4 ou 5,
**caractérisé en ce que**
un résidu Arg est présent en position 47.

7. Polypeptide selon la revendication 6,
**caractérisé en ce que**
il y a la séquence Val⁴⁴-Thr⁴⁵-Ala⁴⁶-Arg⁴⁷ dans la zone des positions d'acides aminés 44 à 47.

8. Polypeptide selon la revendication 6,
**caractérisé en ce que**
il y a la séquence Ile⁴⁴-Gly⁴⁵-Ala⁴⁶-Arg⁴⁷ dans la zone des positions d'acides aminés 44 à 47.

9. Polypeptide selon l'une quelconque des revendications 1 à 8, **caractérisé en ce que**
l'affinité de liaison pour les ligands peptidiques est telle qu'une élution de compétition avec des ligands de streptavidine, choisis parmi la biotine, l'iminobiotine, l'acide lipoïque, la desthiobiotine, la diaminobiotine, le HABA et/ou le diméthyl-HABA puisse se faire.

10. Polypeptide selon l'une quelconque des revendications 1 à 9, **caractérisé en ce que**
la constante d'affinité du complexe constitué d'un polypeptide et d'un ligand peptidique, par rapport à la séquence AWRHPQFGG, est > 2,7·10⁴ M⁻¹ et, par rapport à la séquence WSHPQFEK, > 1,4·10⁴ M⁻¹.

11. Polypeptide selon l'une quelconque des revendications 1 à 10, **caractérisé en ce que**
il porte au moins un groupement de marquage.

12. Acide nucléique,
**caractérisé en ce que**
il comprend une séquence codant pour la mutéine de la streptavidine selon l'une quelconque des revendications 1 à 10.

13. Vecteur, comprenant au moins une copie d'un acide nucléique selon la revendication 12 dans un environnement fonctionnel au plan opérationnel.

14. Cellule,
**caractérisée en ce que**
elle fait l'objet d'une transformation ou d'une transfection avec un vecteur selon la revendication 13.

15. Procédé de fabrication d'une mutéine de la streptavidine selon l'une quelconque des revendications 1 à 10, **caractérisé par** les étapes suivantes :
(a) transformation d'une cellule hôte appropriée avec un vecteur, qui comprend un acide nucléique codant pour la mutéine de la streptavidine,
(b) mise en culture de la cellule hôte dans des conditions permettant une expression de la mutéine de la streptavidine, et
(c) obtention du polypeptide.

16. Procédé selon la revendication 15,
dans lequel l'étape (c) comprend la lyse des cellules hôtes, l'obtention de la mutéine de la streptavidine sous la forme de corps d'inclusion et la renaturation de la mutéine de la streptavidine.

17. Procédé selon la revendication 15, dans lequel l'expression réalisée à l'étape (b) comprend l'expression d'une streptavidine portant un peptide signal et sa sécrétion dans le périplasme de la cellule hôte ou dans le milieu de culture.

18. Procédé selon l'une quelconque des revendications 15 à 17, dans lequel la cellule hôte est E. coli.

19. Utilisation d'une mutéine de la streptavidine selon l'une quelconque des revendications 1 à 11 dans un procédé pour isoler, purifier ou déterminer une protéine, qui est fusionnée avec une séquence peptidique de formule Trp-X-His-Pro-Gln-Phe-Y-Z, où X représente n'importe quel acide aminé et Y et Z représentent tous deux le résidu Gly ou Y représente le résidu Glu et Z représente le résidu Arg ou Lys, dans laquelle on met en contact un liquide contenant la protéine à isoler, à purifier ou à déterminer avec la mutéine de la streptavidine dans des conditions appropriées pour assurer une liaison de la séquence peptidique à la mutéine de la streptavidine, on sépare le complexe obtenu du liquide et on libère ou on détermine la protéine éventuellement à partir du complexe.

20. Utilisation selon la revendication 19,
**caractérisée en ce que**
la mutéine de la streptavidine est liée à une phase solide ou est capable de s'y lier.

21. Utilisation selon la revendication 19 ou 20,
**caractérisée en ce que,**
pour libérer la protéine de fusion du complexe, on incube le complexe avec une quantité suffisante d'un ligand pour la mutéine de la streptavidine, qui est choisi parmi la biotine et ses dérivés.

22. Utilisation selon la revendication 21,
**caractérisée en ce que**
le dérivé de biotine est la desthiobiotine.

23. Utilisation d'une mutéine de la streptavidine marquée selon la revendication 11 dans un procédé de détermination d'une protéine, qui est fusionnée avec une séquence peptidique de formule Trp-X-His-Pro-Gln-Phe-Y-Z, où X représente n'importe quel acide aminé et Y et Z représentent tous deux un résidu Gly ou Y représente un résidu Glu et Z représente un résidu Arg ou Lys, dans laquelle on met en contact la protéine à déterminer avec la mutéine de la streptavidine marquée dans des conditions appropriées pour assurer une liaison de la séquence peptidique à la mutéine de la streptavidine et on détermine le marquage.

24. Utilisation d'une mutéine de la streptavidine selon l'une quelconque des revendications 1 à 11 pour immobiliser une protéine, qui est fusionnée avec une séquence peptidique de formule Trp-X-His-Pro-Gln-Phe-Y-Z, où X représente n'importe quel acide aminé et Y et Z représentent tous deux un résidu Gly ou Y représente un résidu Glu et Z représente un résidu Arg ou Lys, sur une phase solide.

25. Utilisation selon l'une quelconque des revendications 19 à 23, **caractérisée en ce que**
l'on choisit la séquence peptidique parmi les séquences suivantes : AWRHPQFGG ou WSHPQFEK.

26. Utilisation d'une mutéine de la streptavidine selon l'une quelconque des revendications 1 à 11 pour déterminer ou obtenir des substances qui portent un groupement susceptible de se lier à la streptavidine.

27. Trousse de réactifs pour l'utilisation selon l'une quelconque des revendications 19 à 26, comprenant une mutéine de la streptavidine selon l'une quelconque des revendications 1 à 11, ainsi qu'éventuellement des substances tampons, des adjuvants et des additifs.
